# EUROPEAN PATENT SPECIFICATION

(11) **EP 2 014 707 B1**
(45) Date of publication and mention of the grant of the patent: **23.04.2014**
(21) Application number: 07112305.3
(22) Date of filing: 12.07.2007
(51) Int. Cl.: C08K 5/14

(54) **Modified polymer compositions, modification process and free radical generating agents for i.a. wire and cable applications**
Modifizierte Polymerzusammensetzungen, Modifikationsverfahren und Wirkstoffe zur Erzeugung freier Radikale für I.A.-Draht und Kabelanwendungen
Compositions de polymère modifié, processus de modification et agents de génération radicaux libres pour fil et applications de câble

(43) Date of publication of application: 14.01.2009
(73) Proprietor: Borealis Technology Oy, 06101 Porvoo (FI)
(72) Inventor: Smedberg, Annika, SE-471 61, Myggenäs (SE); Nilsson, Daniel, SE-415 12, Göteborg (SE); Gustafsson, Bill, SE-444 41, Stenungsund (SE)
(74) Representative: Campbell, Neil Boyd

(56) References cited:
- EP-A1- 1 243 614
- WO-A1-02/28946
- WO-A2-2005/066280
- US-A- 4 632 950
- US-A- 5 578 682
- US-B1- 6 172 161

## Description

### Field of invention

The invention relates to a process for modifying a polymer composition, to modified polymer compositions, to an article, preferably wire or cable, comprising said modified polymer composition, to a process for preparing an article, preferably a wire or cable, to the use of said modified polymer in one or more layers of a wire or cable, as well as to a compound for use as a radical generating agent for modifying a polymer composition.

### Background art

It is known to use free radical generating agents for modifying a product, such as a polymer composition via a radical reaction.

Free radical agents are used e.g. to initiate (a) a crosslinking in a polymer, i.a. primarily a formation of interpolymer crosslinks (bridges) by radical reaction, (b) a grafting in a polymer, i.e. introduction of compounds to a polymer chain (to backbone and/or side chains) by radical reaction, and (c) a visbreaking in a polymer, i.e. modification of melt flow rate (MFR) of a polymer by radical reaction. These polymer modifications are well known in the art.

When added to a polymer composition, free radical generating agents act by generating radicals, typically by decomposing to radicals, under conditions which enable the radical formation. The decomposed radicals initiate further radical reactions within a polymer composition. The resulting decomposition products of the free radical generating agent are typically a result of several reactions of the decomposition products of initial radical forming reaction. Said resulting decomposition products typically remain in the modified polymer and may include detrimental, undesired decomposition products.

Peroxides are very common free radical generating agents used i.a. in the polymer industry for said polymer modifications. The resulting decomposition products of peroxides may include volatile by-products. E.g. Dicumylperoxide, which is commonly used peroxide in polymer field, decomposes i.a. to methane, acetophenone and cumylalcohol during the radical formation step, e.g. during crosslinking step. The formed gaseous methane (CH₄) is a flammable and explosive volatile and thus a risk in a working environment.

In wire and cable applications a typical cable comprises at least one conductor surrounded by one or more layers of polymeric materials. In power cables including medium voltage (MV), high voltage (HV) and extra high voltage (EHV) said conductor is surrounded by several layers including an inner semiconductive layer, an insulation layer and an outer semiconductive layer, in that order. The cables are commonly produced by extruding the layers on a conductor. One or more of said layers are then typically crosslinked to improve i.a. deformation resistance at elevated temperatures, as well as mechanical strength and/or chemical resistance, of the layer(s) of the cable. The free radical generating agent, such as peroxide, is typically incorporated to the layer material prior the extrusion of the layer(s) on a conductor. After formation of the layered cable, the cable is then subjected to a crosslinking step to initiate the radical formation and thereby crosslinking reaction. The decomposition products of the free radical forming agent remain mostly captured within the cable layer after crosslinking. This causes problems in view of the cable manufacturing process as well as in view of the quality of the final cable.

Accordingly, after crosslinking the cable must be cooled with high care in order to prevent the gaseous volatile decomposition products like methane to form voids within the polymer layer. These voids have typically an average diameter of between 10 to 100 µm. Partial discharges can take place in such voids within a cable that is subjected to an electrical field and thereby reduce the electrical strength of the cable.

The MV, HV and EHV power cables must have high layer quality in terms of safety during installation and in end use thereof In service volatile decomposition products of a crosslinking step in cable can create a gas pressure and thus cause defects in the shielding and in the joints. E.g. when a cable is equipped with a metal barrier, then the gaseous products can exert a pressure, especially on the joints and terminations, whereby a system failure may occur.

Due to above reasons the volatile decomposition products, such as methane in case dicumylperoxide is used, are conventionally reduced in minimum or removed after crosslinking and cooling step. Such removal step is generally known as degassing step.
The degassing step is a time and energy consuming and thus costly operation in a cable manufacturing process. Degassing requires large heated chambers which must be well ventilated to avoid the build-up of e.g. flammable methane and ethane. The cable, typically wound to cable drums, is normally held in said degassing in elevated temperature in the range of 50-80°C, e.g. 60-70°C, for lengthy time periods. In required temperatures thermal expansion and softening of the insulation can occur and lead to undue deformation of the formed cable layers resulting directly to failures of the cable. The degassing of HV and EHV cables with high cable weight needs thus often be carried out in decreased temperatures.

Accordingly, there is a need to find new solutions to overcome the prior art problems.

### Objects of the invention

One of the objects of the present invention is to provide an alternative process for modifying a polymer composition by using a free radical generating agent with superior properties.

A further object of the invention is to provide a modified polymer composition exhibiting excellent properties, such as a high quality, useful for many end applications of polymers, i.a. for wire and cable applications.

Another object of the invention is to provide an article produced by said modified polymer composition, such as a cable which comprises one or more layers comprising said modified polymer composition, which article has highly advantageous properties, such as a high quality and superior processability properties.

A further object of the invention is to provide a process for producing an article using said modified polymer composition, preferably a cable, as defined above, which process enables the preparation of high quality products with shorter production time and/or lower energy consumption.

A still further object of the invention is to provide a compound for use as a free radical generating agent which enables to modify polymers in an environmentally friendly and/or healthy manner and to obtain modified polymers with high quality. Also the use of said compound as free radical generating agent is provided.

The invention and further objects thereof are described and defined in details below.

### Description of the invention

The objects of the invention are solved by the compounds, polymer compositions, end products and process as defined i.a. in below claims.

### Compounds of the invention

Accordingly, the invention provides a compound which is an organic peroxide of formula (I) 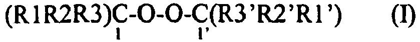 wherein
- R1 and R1' are each independently substituted or unsubstituted saturated or partially unsaturated hydrocarbyl; or substituted or unsubstituted aromatic hydrocarbyl;
- wherein each of said substituted or unsubstituted saturated or partially unsaturated hydrocarbyl or aromatic hydrocarbyl optionally comprises one or more heteroatoms;
- wherein said substituted or unsubstituted saturated or partially unsaturated hydrocarbyl include straight or branched chain saturated or partially unsaturated hydrocarbyls; straight or branched chain saturated or partially unsaturated hydrocarbyls which bear saturated or partially unsaturated cyclic hydrocarbyl; and saturated or partially unsaturated cyclic hydrocarbyls;
- wherein each of said aromatic hydrocarbyl and said saturated or partially unsaturated cyclic hydrocarbyl is independently a monocyclic or multicyclic ring system; and
- wherein said substituted saturated or partially unsaturated hydrocarbyl or substituted aromatic hydrocarbyl comprises independently 1 to 4 substituents selected from a functional group, saturated or partially unsaturated hydrocarbyl optionally bearing a functional group; or aromatic hydrocarbyl optionally bearing a functional group;
- R2, R2', R3 and R3' are each independently H, substituted or unsubstituted saturated or partially unsaturated hydrocarbyl; or substituted or unsubstituted aromatic hydrocarbyl;
- wherein each of said substituted or unsubstituted saturated or partially unsaturated hydrocarbyl or aromatic hydrocarbyl optionally comprises one or more heteroatoms;
- wherein said substituted or unsubstituted saturated or partially unsaturated hydrocarbyl include straight or branched chain saturated or partially unsaturated hydrocarbyls; straight or branched chain saturated or partially unsaturated hydrocarbyls which bear saturated or partially unsaturated cyclic hydrocarbyl; and saturated or partially unsaturated cyclic hydrocarbyls;
- wherein each of said aromatic hydrocarbyl and said saturated or partially unsaturated cyclic hydrocarbyl is independently a monocyclic or multicyclic ring system; and
- wherein said substituted saturated or partially unsaturated hydrocarbyl or substituted aromatic hydrocarbyl comprises independently 1 to 4 substituents selected from a functional group, saturated or partially unsaturated hydrocarbyl optionally bearing a functional group; or aromatic hydrocarbyl optionally bearing a functional group; or
- R2 and R3 together with the carbon atom (C1) to which they are attached form unsubstituted or substituted saturated or partially unsaturated carbocyclic ring moiety of 3 to 14 C-atoms; unsubstituted or substituted saturated or partially unsaturated heteroring moiety of 3 to 14 ring atoms comprising 1 to 6 heteroatoms selected from O, N, P, S or Si; or unsubstituted or substituted aromatic ring moiety of 3 to 14 C-atoms optionally comprising 1 to 4 heteroatoms;
- wherein said carbocyclic ring, heteroring or aromatic ring system is optionally fused with another ring system having 4 to 14 ring atoms; and
- wherein said substituted carbocyclic ring, heteroring or aromatic ring system comprises 1 to 4 substituents selected independently from a functional group or saturated or partially unsaturated hydrocarbyl optionally bearing a functional group or aromatic hydrocarbyl optionally bearing a functional group; or
- R2' and R3' together wi th the carbon atom (C1') to which they are attached form unsubstituted or substituted saturated or partially unsaturated carbocyclic ring moiety of 3 to 14 C-atoms; unsubstituted or substituted saturated or partially unsaturated heteroring moiety of 3 to 14 ring atoms comprising 1 to 6 heteroatoms selected from O, N, P, S or Si; or unsubstituted or substituted aromatic ring moiety of 3 to 14 C-atoms optionally comprising 1 to 4 heteroatoms;
- wherein said carbocyclic ring, heteroring or aromatic ring system is optionally fused with another ring system having 4 to 14 ring atoms; and
- wherein said substituted carbocyclic ring, heteroring or aromatic ring system comprises 1 to 4 substituents selected independently from a functional group or a saturated or partially unsaturated hydrocarbyl optionally bearing a functional group or aromatic hydrocarbyl optionally bearing a functional group; or
- R2 and R2' form together a bivalent substituted or unsubstituted saturated or partly unsaturated hydrocarbyl optionally containing 1 to 4 heteroatoms, wherein R2 is linked to C1 and R2' to C1', respectively, forming together with -C1-O-O-C1'-substituted or unsubstituted saturated or partially unsaturated carbocyclic ring moiety of 3 to 14 C-atoms comprising optionally, in addition to said at least two O atoms, 1 to 4 further heteroatoms; wherein said carbocyclic ring or heteroring system is optionally fused with another ring system having 4-14 ring atoms;
   and functional derivatives thereof;
- with a proviso that at least two of R1, R2 and R3, and at least two of R1', R2' and R3', respectively, are other than H or methyl.

Generally, in below definitions the given values in ppm for methane and/or other volatile content are determined by gas chromatography from the obtained crosslinked polymer composition as such or from a crosslinked cable layer, depending on the definition, according to a method as described below under "GC-analysis protocol". Without limiting to any theory, the terms "a decomposition product(s) thereof" or "a decomposition product of a free radical generating step" etc. as used above and below mean herein a by-product(s) formed during a free radical generating step, e.g. crosslinking step, and possibly also during the cooling step, by initiation of the free radical generating agent, as well known in the art. As an example methane may be one decomposition product which is an undesired decomposition product of the invention. Further decomposition products are specified below, which may not be desired in various embodiments of the invention.

The term "without resulting in CH₄ as a decomposition product thereof" means that a compound of the present invention generates no methane, or in alternative terms does not decompose to undesired volatile CH₄ by-product during a radical formation step in an industrial process.

The solution of the invention provides a new principal which is surprising and unobvious, since in the prior art there have not been any teaching or any indication to modify the free radical generating agent in order to avoid formation of CH₄ as a decomposing product during the free radical formation step in an industrial process.
E.g. in crosslinking applications the prior art has proposed merely solutions relating to balance the amount of free radical generating agent and the needed degree of crosslinking.

In one embodiment said compound of the invention results in a CH₄ content of less than 300 ppm (weight), preferably of less than 200 ppm (weight), preferably of less than 100 ppm (weight), more preferably of 0 to 50 ppm (weight), even more preferably is without resulting to CH₄, as a decomposition product thereof.

In one embodiment said compound of the invention results in reduced amount of or preferably does not decompose to low molecule compounds selected from (C1-C3)alkanes when generating free radicals in industrial applications.

In another embodiment of the invention it is advantageous that said compound as a free radical generating agent results in reduced amount of or preferably is without (C1-C4)alkanes as decomposition products thereof when generating free radicals in industrial applications.

In embodiments, wherein very high quality is required for the products modified by using a free radical agent, then it is preferable that said compound results in reduced amount of or is preferably without (C1-C6)alkanes as decomposition products thereof during a free radical forming step in an industrial process.

The term "a free radical generating agent" is defined herein above or below to be any compound capable to generate radicals in industrial applications, e.g. which can initiate a modification reaction in a polymer, such as a crosslinking, grafting or visbreaking reaction in a polymer, which all embodiments will be further discussed below.

The compounds of the invention are free radical generating agents which are organic peroxides of formula (I) 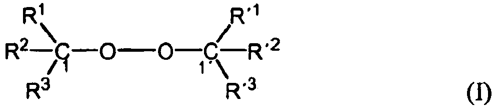 as hereinbefore defined.

The compounds of formula (I), form an independent group of compounds of the invention, which all groups have the same unitary feature, i.e. a reduced amount of a volatile decomposition product over the prior art. All the four groups of compounds of the invention can be made dependent on each other in any combination, and in any order.

Preferably, the compound of formula (I) as defined above results in CH₄ content of less than 300 ppm (weight), preferably of less than 200 ppm (weight), preferably less than 100 ppm (weight), more preferably is without CH₄ as a decomposition product thereof, during an industrial process for generating free radicals, e.g. during a modification step of a polymer composition.

The terms used for defining the compounds of formula (I) are well known in the organic chemistry. E.g. in moieties defined in formula (I):
The expression "partially unsaturated" means that the moiety may comprise one or more double or triple bonds and include alkenyl radicals comprising at least one double bond and alkynyl radicals comprising at least one triple bond. In case of "partially unsaturated cyclic hydrocarbyl" there can be one or more double bonds in the ring systems meaning that the ring is non-aromatic to differentiate said "partially unsaturated" ring moieties from "aromatic rings" such as phenyl or pyridyl radicals.

"Hetero atoms" present in the moieties are selected from N, O, P, S or Si.

The expression "monocyclic" includes monocyclic ring systems, such as cyclopentyl, cyclohexyl, cycloheptyl or phenyl radical,
The expression "multicyclic" in turn means herein fused ring systems, such as naphthyl radical.

The term "functional group" as a substituent is well known expression and includes i.a. -OH, -NR₂, wherein each R is independently H or (C1-C12)alkyl; -O-O-, COR, wherein R is i.a. H, (C1-C12)alkyl or -NR₂, wherein each R is as defined for -NR₂, COOR, wherein R is as defined for -COR.

The term "optional" means that may or may not be present, e.g. "optionally substituted" cover options wherein a substituent is present and wherein no substituent is present. The term "unsubstituted" naturally means that no substituent is present.

The term "functional derivative" includes i.a. esters and salts of compounds of formula (I).

It is naturally understood herein, that the above groups of compound for use as a free radical generating agent defined in terms of the decomposition product thereof and the below following subgroups of the compound of formula (I) of the invention represent some preferable embodiments and variants of the invention. It is also understood that said below subgroups further specify the substituents given above in formula (I). Above and below subgroups are thus generalisable and can be in any combination to define further subgroups within the broadest scope of compounds of formula (I) of the invention. Moreover said above generally defined compounds of first, second and third group and said subgroups thereof, and the general definition for compounds of formula (I), as well as said subgroup thereof, can be combined in any combination in their uses for modifying polymers, to modification methods, to modified polymers and to articles comprising said modified polymers, as well as to preparation process thereof, which all aspects of the invention are discussed below.

In a subgroup (1) of the compound of formula (I) as defined above, R2 and R3 together with carbon atom (C1) they are attached to form an optionally substituted carbocyclic ring moiety of 3 to 12 ring C-atoms or an optionally substituted heteroring moiety of 3 to 12 ring atoms containing 1 to 6, preferably 1 to 4, heteroatoms selected from O, N, P, S or Si, and wherein said carbocyclic or heterocyclic ring system is optionally fused with another ring system having 4 to 14 ring atoms, preferably R2 and R3 together with carbon atom (C1) form a (C3-C12)carbocyclic ring moiety.

In a subgroup (2) of the compound of formula (I) as defined above, R2' and R3' together with carbon atom (C1') they are attached to form an optionally substituted carbocyclic ring moiety of 3 to 12 ring C-atoms or an optionally substituted heteroring moiety of 3 to 12 ring atoms containing 1 to 6, preferably 1 to 4, heteroatoms selected from O, N, P, S or Si, and wherein said carbocyclic or heterocyclic ring system is optionally fused with another ring system having 4 tol4 ring atoms, preferably R2' and R3' together with carbon atom (C1') form a (C3-C12)carbocyclic ring moiety.

In a subgroup (3) of the compound of formula (I) as defined above, R2 and R3 together with the carbon atom (C1) they are attached to form an optionally substituted, saturated or partially unsaturated mono- or bicyclic (C4-C14)carbocyclic ring, preferably unsubstituted saturated monocyclic (C5-C8)carbocyclic ring, such as cyclopentyl, cyclohexyl or cylcoheptyl, preferably cyclohexyl.

In a subgroup (4) of the compound of formula (I) as defined above, R2' and R3' together with the carbon atom (C1') they are attached to form an optionally substituted, saturated or partially unsaturated mono- or bicyclic (C4-C14)carbocyclic ring, preferably unsubstituted saturated monocyclic (C5-C8)carbocyclic ring, such as cyclopentyl, cyclohexyl or cylcoheptyl, preferably cyclohexyl.

In a subgroup (5) of the compound of formula (I) as defined above, R2 and R3 together with the carbon atom (C1) they are attached to form a ring system as defined in subgroup (3) and R2' and R3' together with the carbon atom (C1') they are attached to form a ring system as defined in subgroup (4), whereby the ring system formed by R2' and R3' together with the carbon atom (C1') they are attached to is identical to the ring system formed by R2 and R3 together with the carbon atom (C1) they are attached to.

In a subgroup (6) of the compound of formula (I) as defined above, R1, R2, R3, R1', R2' and R3' each independently is optionally substituted mono- or multicyclic (C5-C14)aryl, optionally substituted mono- or multicyclic (C5-C14)heteroaryl, optionally substituted mono- or multicyclic (C4-C14)cycloalkyl, optionally substituted mono- or multicyclic (C4-C14)heterocyclyl, optionally substituted straight or branched chain (C1-C50)alkyl, preferably straight chain (C1-C30)alkyl, optionally substituted straight or branched chain, preferably straight chain, (C1-C50)alkenyl or ptionally substituted straight or branched chain, preferably straight chain, (C1-C50)alkynyl, preferably straight chain (C1-C30)alkenyl or straight chain (C1-C30)alkynyl, optionally substituted straight or branched chain (C1-C50)heteroalkyl comprising 1 to 4 heteroatoms selected from N, O, S or Si.

In a subgroup (7) of the compound of formula (I) as defined above, R2, R2', R3 and R3' are each independently selected from unsubstituted straight chain (C1-C50)alkyl, preferably (C1-C30)alkyl, more preferably (C1-C20)alkyl, such as hexyl, heptyl, octyl, decyl, undecyl, docedyl, preferably decyl

In a subgroup (8) of the compound of formula (I) as defined above, R2 and R2' each represents same radical and, respectively, R3 and R3' each represents same radical.

In a subgroup (9) of the compound of formula (I) as defined above, R2 and R2' are same and each represents methyl.

In a subgroup (10) of the compound of formula (I) as defined above, R2 and R2' are same and each represents (C6-C30)alkyl.

In a subgroup (11) of the compound of formula (I) as defined above, R3 and R3' are same and each represents (C6-C30)alkyl.

In a subgroup (12) of the compound of formula (I) as defined above R1 and R1' are same or different, preferably same, and each represents optionally substituted, saturated or partially unsaturated cyclic hydrocarbyl of 5 to 14 ring atoms optionally containing 1 to 4 heteroring atoms selected from N, O, P, S or Si, or optionally substituted mono- or multicyclic (C5-C14)aryl, preferably unsubstituted monocyclic (C5-C7)aryl.

In a subgroup (13) of the compound of formula (I) as defined above, R1 and R1' are same or different, preferably same, and each represents optionally substituted branched or straight chain, preferably unsubstituted straight chain, (C6-C30)alkyl or methyl.

In a subgroup (14) of the compounds of formula (I) are as defined above, with a further proviso that at least two of R1, R2 and R3, and at least two of R1', R2' and R3', respectively, are other than H, methyl, iso-butyl or tert-butyl.

In a subgroup (15) of the compounds of formula (I) are as defined above, with a further proviso that at least two of R1, R2 and R3, and at least two of R1', R2' and R3', respectively, are other than H, methyl, ethyl, 1-propyl, isopropyl, 1-butyl, iso-butyl or tert-butyl.

In a subgroup (16) of the compounds of formula (I) as defined above, R1, R2 and R3, and R1', R2' and R3', respectively, are each other than CH₄, preferably other than straight or branched chain saturated or partially unsaturated (C1-C3)hydrocarbyl, more preferably other than straight or branched chain saturated or partially unsaturated (C 1-C4)hydrocarbyl,
Subgroups (14), (15) and (16) are useful for embodiments wherein very high purity products, e.g. polymers, are desirable after the modification step with compound (I).

In one preferable subgroup (Ia) of compounds of formula (I) as defined above, R1 and R1' are both same and represent an optionally substituted, preferably unsubstituted, monocyclic (C5-C7)aryl; R2 and R3 form together with C1 atom, wherein they are attached to, an optionally substituted, saturated or partially unsaturated mono- or bicyclic (C4-C14)carbocyclic ring, preferably unsubstituted saturated monocyclic (C5-C8)carbocyclic ring, and R2' and R3' form together with the carbon atom (C1'), wherein they are attached to, an optionally substituted, saturated or partially unsaturated mono- or bicyclic (C4-C14)carbocyclic ring, preferably unsubstituted saturated monocyclic (C5-C8)carbocyclic ring; whereby the ring system formed by R2 and R3 together with C1 wherein they are attached to is identical to a ring system formed by R2' and R3' together with C1' wherein they are attached to.

In another preferable subgroup (Ib) of compounds of formula (I) as defined above, R1 and R1' are both same and represent an optionally substituted, preferably unsubstituted, monocyclic (C5-C7)aryl; R2 and R2' are same and are both methyl; and R3 and R3' are same and are both optionally substituted branched or straight chain, preferably unsubstituted straight chain, (C6-C50)alkyl, more preferably unsubstituted straight chain (C6-C30)alkyl, such as (C6-C20)alkyl.

In a further preferable subgroup (Ic) of compounds of formula (I) as defined above, R1 and R1' are both same and represent an optionally substituted, preferably unsubstituted, monocyclic (C5-C7)aryl; R2 and R2' are same and are both optionally substituted branched or straight chain, preferably unsubstituted straight chain, (C6-C50)alkyl, more preferably unsubstituted straight chain (C6-C30)alkyl, such as (C6-C20)alkyl; and R3 and R3' are same and are both optionally substituted branched or straight chain, preferably unsubstituted straight chain, (C6-C50)alkyl, more preferably unsubstituted straight chain (C6-C30)alkyl, such as (C6-C20)alkyl.

One preferable compound of formula (I) is Di-(1-phenylcyclohexyl) peroxide (formula Ia):

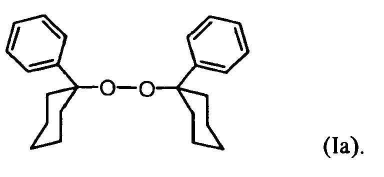

One preferable compound of formula (I) is Di-(1-methyl-1-phenylundecyl) peroxide (formula Ib):

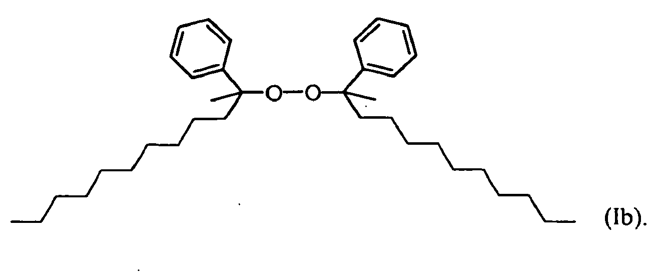

One preferable compound of formula (I) is Di-(1-decyl-1-phenylundecyl) peroxide (formula Ic):

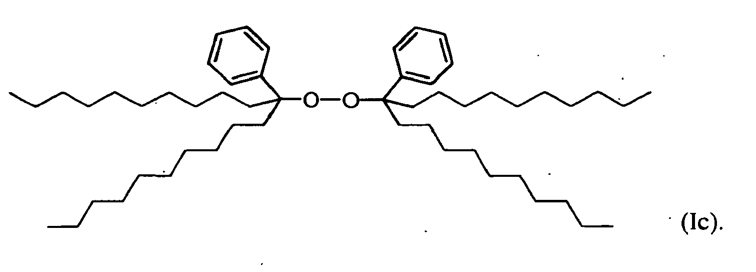

Said specific compounds of formula (Ib) and (Ic) are novel as such.
Thus the invention is directed to the compound of formula (Ib) as defined above. The invention is further directed to the compound of formula (Ic) as defined above.

### Preparation of the compounds of formula (I)

The compounds of the invention include novel and known compounds. The use of the known compounds as a free radical generating agent, preferably for modifying a polymer composition, is novel. Thus said known compounds may be commercially available. Alternatively, the compounds of the invention can be prepared according to or analogously to known methods described in the chemical literature.

As an example, the compounds (I) as defined above can be prepared according to the following scheme 1 using known procedures which are described in a literature and well known for a skilled person in the art.

Peroxides of formula (I) as defined above can be prepared in several known manner, and more specifically tertiary peroxides can be prepared from the corresponding tertiary alcohols under acidic conditions to give compound (I). The alcohols are either commercially available, or can be prepared from a suitable ketone combined with a organometallic reagent, more specifically a Grignard (RMgX, where X is an halogen) or organolithium (RLi) reagent.

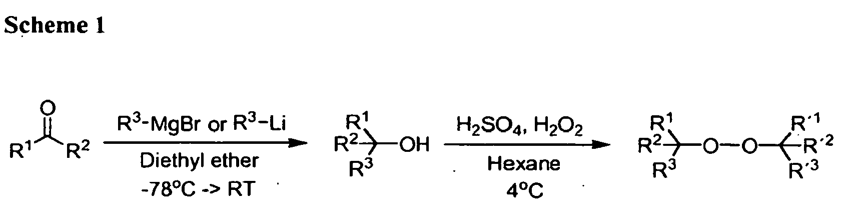

References to synthetic methods are as follows:
1) Milas, N.A., Surgenor, D.M., J. Am. Chem. Soc., 643-644, 1946
2) Hey, D.H., Stirling, C.J.M., Williams, G.H, J. Chem. Soc., 1054-1058, 1957
3) Organic Synthesis, Smith, M.B., The McGraw-Hill Companies Inc., 2002

The formed tertiary alcohol and corresponding peroxide can be purified by removing the solvent *in vacuo* and purifying the residue by any of the methods known to those skilled in the art, such as crystallization.

Accordingly, the present invention reduces or minimises the fire, explosion and healthy risks in an working environment caused by the use of free radical generating agents compared to the prior art.

### End uses and end applications of the invention

### I. Modification method of polymers

The invention is directed to the use of Compound of the invention as a free radical generating agent for modifying polymers by radical formation. Also a process for modifying polymers by radical reaction using a free radical generating agent is provided, wherein said free radical generating agent is Compound of the invention.

### 1. Crosslinking of polymers

One preferable embodiment of said modification method of polymers of the invention is crosslinking of polymers by radical reaction using one or more free radical generating agents, wherein at least one said free radical generating agent is Compound of the invention as defined above.

The term "crosslinking" is well known and commonly used in the polymer field and means forming, primarily, of interpolymer crosslinks (bridges) via radical reaction.

In principle, the polymers usable in the crosslinking process of the present invention are not limited and can be polymers of any type.

In one preferable embodiment, said crosslinkable polymer is a polyolefin which can be a homopolymer of an olefin or a copolymer of an olefin with one or more comonomers.

As one preferable group of crosslinkable polyolefins includes homopolymer of ethylene or copolymer of ethylene with one or more comonomers, such as 1) a branched polyethylene homo- or copolymer produced in high pressure by radical polymerisation and well known as low density polyethylene (LDPE) homo or copolymer or 2) a linear polyethylene homo- or copolymer produced by low pressure polymerisation using a coordination catalyst, such as well known linear very low density polyethylene, linear low density polyethylene (LLDPE), medium density polyethylene (MDPE) or high density polyethylene (HDPE), 3) polypropylene polymers, including homopolymers and random polymers of polypropylene and heterophasic copolymer of propylene, or 4) polybutylene polymers, as non-limiting examples only.

One preferable group of crosslinkable ethylene polymers is 1) LDPE homopolymer or LDPE copolymer with one or more comonomers including C3 or higher alphaolefin copolymer(s), polar comonomers and comonomers with at least two double bonds, such as diene comonomers. High pressure polymerisation is a well known technology in the polymer field and can be effected in a tubular or an autoclave reactor, preferably, in a tubular reactor. Further details about high pressure radical polymerisation are given in WO 93/08222.
In one preferable embodiment of crosslinkable LDPE is LDPE copolymer of ethylene with a polar group containing comonomer(s) and, optionally, with other comonomer(s). As examples of comonomers having polar groups may be mentioned the following: (a) vinyl carboxylate esters, such as vinyl acetate and vinyl pivalate, (b) (meth)acrylates, such as methyl(meth)acrylate, ethyl(meth)acrylate, butyl(meth)acrylate and hydroxyethyl(meth)acrylate, (c) olefinically unsaturated carboxylic acids, such as (meth)acrylic acid, maleic acid and fumaric acid, (d) (meth)acrylic acid derivatives, such as (meth)acrylonitrile and (meth)acrylic amide, and (e) vinyl ethers, such as vinyl methyl ether and vinyl phenyl ether.

Amongst these comonomers, vinyl esters of monocarboxylic acids having 1 to 4 carbon atoms, such as vinyl acetate, and (meth)acrylates of alcohols having 1 to 4 carbon atoms, such as methyl (meth)acrylate, are preferred.
Especially preferred comonomers are butyl acrylate, ethyl acrylate and methyl acrylate.
The term "(meth)acrylic acid" and "(meth)acrylate" are intended to embrace both acrylic acid and methacrylic acid and, respectively "methacrylate" and "acrylate".

Moreover, 1) said crosslinkable LDPE suitable for the present invention may be an unsaturated polyolefin which is prepared by copolymerising at least one olefin monomer with at least one polyunsaturated comonomer. Such polymer are well known and described e.g. in WO 93/08222, EP1695996 or W02006/131266. In addition to the vinyl groups originating from the polyunsaturated comonomer, the content of vinyl groups may also be originated, alone or additionally, from a chain transfer agent which introduces vinyl groups, such as propylene.

Also 2) linear ethylene polymers prepared using said low pressure polymerisation are very suitable for the crosslinking of the invention. As an example LLDPE, MDPE and HDPE polymers can be mentioned. They can be produced in a known manner in a single or multistage processes using one or more of e.g. Ziegler-Natta catalysts, single site catalysts, including metallocenes and non-metallocenes, and Cr-catalysts. All said catalysts are very well known in the field. The multistage process includes any combinations of polymerisation processes, such as slurry polymerisation, solution polymerisation, gas phase polymerisation, or any combinations thereof, in any order.

Generally, crosslinkable polymers that are usable in the present invention include any known polymers, e.g. commercially available polymers, or they can prepared in a known manner according to or analogously to polymerisation process described in the literatured. Naturally any mixtures of polymers can also be used.

The amount of the Compound of the invention as a free radical generating agent used for the crosslinking is not critical and can vary depending on the desired crosslinking degree and the type of the crosslinkable polymer. As an example only, the amount of said free radical generating agent of the invention may be less than 10.0 wt%, less than 6.0 wt%, less than 5.0 wt%, less than 3.5 wt%, e.g. between 0.1 to 3.0 wt%, such as 0.2 to 2.6 wt%, based on the weight of the crosslinkable polymer composition, depending i.a. on the molecular weight of Compound and the desired degree of crosslinking.
The crosslinking may be carried out in a known manner, typically in elevated temperature, such as 140°C or more, preferably 150°C or more. And said step may be effected under atmospheric or typically slightly pressurised conditions, e.g. up to 20 bar, e.g. up to about 13 bar, pressure.

### 2. Grafting of polymers

Another embodiment of the modification method of the invention is a grafting of a polymer by radical reaction using a free radical generating agent, wherein said free radical generating agent is Compound of the invention.

The "grafting of a polymer" is a well known modification method and means introducing compounds to the polymer chain (backbone and/or side chain) by radical reaction. Typically the compounds to be incorporated by grafting are unsaturated. Such grafting process are described in the literature, e.g. in US 3,646,155 and US 4,117,195.

In one preferable embodiment of grafting of the invention the polymer is grafted by an unsaturated silane compound represented by the formula R¹SiR²_{q}Y_{3-q} (I)
wherein
R¹ is an ethylenically unsaturated hydrocarbyl, hydrocarbyloxy or (meth)acryloxy hydrocarbyl group,
R² is an aliphatic saturated hydrocarbyl group,
Y which may be the same or different, is a hydrolysable organic group and
q is 0, 1 or 2.
Commonly used compounds are vinyl trimethoxysilane, vinyl
bismethoxyethoxysilane, vinyl triethoxysilane, gamma-(meth)acryloxypropyltrimethoxysilane, gamma(meth)acryloxypropyltriethoxysilane, and vinyl triacetoxysilane.

The amount of the compound to be grafted, e.g. of said silane compound is not critical and depends on the desired grafting degree. As an example, a silane compound may be grafted in an amount of. 0.001 to 15 wt%, preferably 0.01 to 10 wt%, such as 0.01 to 5 wt% based on the weight of the grafted polymer composition to be grafted.

The amount of Compound of the invention as a free radical generating agent is not critical and depends on the desired grafting degree. As an example only, the amounts exemplified above under "1.Crosslinking of polymers" can be used.

The invention further provides a grafted polymer composition obtainable by a grafting process of the invention as defined above. Said grafted polymer composition may be further treated. Silane-grafted polymers may e.g. be further crosslinked, in a well known manner by hydrolysing said silane groups and subsequently condensing using a silanol condensation catalyst. As such catalysts e.g. known tin based catalyst and aromatic organic sulphonic acids can be used. Also the silane-grafted and crosslinked products are within the scope of the invention.

### 3. Visbreaking of polymers

A further embodiment of the modification method of the invention is a visbreaking of a polymer by radical reaction using a free radical generating agent, wherein said free radical generating agent is Compound of the invention.

The "visbreaking of a polymer" is a well known modification method for modifying the melt flow rate (MFR) of a polymer.

One preferable polymer for visbreaking are homopolymers of propylene, random copolymers of propylene or heterophasic copolymers of propylene. Suitable homo, random and heterophasic polypropylenes (PP) may be produced using common polymerisation processes known in the art. Also the polypropylene polymer may be produced in a single- or multistage process of propylene or propylene and one or more comonomers, such as ethylene or higher alpha olefin. The polymerisation can be effected as bulk polymerisation, gas phase polymerisation, slurry polymerisation, solution polymerisation or any combinations thereof, in any order, using conventional catalysts including Ziegler-Natta, single site, such as metallocene or non-metallocene, catalysts. In case of heterophasic copolymer of polypropylene the matrix of homo or random copolymer can be produced e.g. in a single stage or as a multistage process described above and the elastomeric (rubber) part of the propylene copolymer can be produced as a in-situ polymerisation e.g. in a separate reactor, e.g. gas phase reactor in the presence of the matrix polymer produced in the previous stage(s). Alternatively the elastomeric part of propylene can be incorporated to the matrix phase material by compounding. The heterophasic copolymer of polypropylene thus comprises a matrix phase of homo- or copolymer of polypropylene and an elastomeric propylene copolymer dispersed in the matrix.

The amount of free radical generating agent used for visbreaking is not critical and depends on the level of desired MFR modification. Visbreaking can be carried out in known manner using very well known process and conditions documented in the literature.

The invention further provides a visbroken polymer composition obtainable by the visbreaking process of the invention.

### II. Polymer composition

The invention thus provides also a polymer composition comprising a free radical generating agent which is Compound of the invention as defined above.

The amount of the Compound of the invention can naturally vary depending on the desired modification method. Examples of the amounts are given e.g. above under "1.Crosslinking of polymers". Moreover, the polymer composition of the invention may additionally, comprise further free radical generating agent(s), such as another Compound of the invention.
Furthermore, polymers suitable for the polymer composition of the invention are not limited and include e.g. polymers 1) to 4), preferably 1) LDPE homo and copolymers, as described above under "1.Crosslinking of polymers".

The polymer composition of the invention can be in a well known powder or pellet form, or in a form of polymer melt. Polymer powder of the invention may i.a. be obtained directly from the polymerization process, optionally further processed, e.g. sieved, and/or optionally treated or mixed with further components.

The polymer pellets of the invention can be produced in a well known manner. In one process for forming the pellets of the invention the polymer powder or melt obtained from a polymerization process, or alternatively polymer pellets, may optionally be mixed with other components and pelletised e.g. by extrusion in a known pelletising equipment. The Compound of the invention may be added 1) to a mixture of a polymer composition prior to pelletising step or 2) after pelletising step by adding Compound of the invention to the preformed pellets by mixing and/or impregnating, optionally in a carrier medium, to obtain the polymer pellets of the invention.

Alternatively, Compound of the invention can added to the polymer powder or polymer pellets directly in a production line of an end product, such as cable production line. Addition can be effected in a mixing step preceding the end product formation step or during the end product formation step, e.g. cable extrusion step.

Such addition can be effect to the polymer composition in powder or pellet form or to a melt mixture of said powder or pellets which mixture may optionally contain further components.

Accordingly the invention provides a polymer powder, polymer pellets or a polymer melt comprising a polymer composition and a free radical forming agent, wherein said free radical forming agent is Compound of the invention.

In one preferable embodiment of the pellets or powder of the invention, said pellets or powder are in a package, such as a container including boxes and bags. Such containers can be supplied for further use. E.g. an end producer, e.g. a cable producer, can then use the pellets of the invention as such for polymer modification step without need to add any free radical generating agent.

Moreover, the polymer composition of the invention may further contain other components, such as other polymers as mentioned above, or additives, such as stabilizers.

In one preferable embodiment of the polymer composition of the invention said polymer composition further comprises additives, such as one or more of antioxidants, stabilisers, processing aids, scorch retardants, crosslinking boosters or water tree retarardants, or any mixtures thereof. As antioxidant, sterically hindered or semi-hindered phenols, optionally substituted with functional group(s), aromatic amines, aliphatic sterically hindered amines, organic phosphates, thio compounds, and mixtures thereof, can be mentioned. Typical cross-linking boosters may include compounds having a vinyl or an allyl group, e.g. triallylcyanurate, triallylisocyanurate, and di-, tri- or tetra-acrylates. As further additives, flame retardant additives, acid scavengers, fillers, such as carbon black, and voltage stabilizers can be mentioned. All the above mentioned additives are well known in polymer field. Such compositions are very useful for wire and cable applications, such as for cables of the invention discussed below.

The invention further provides (a) a process for crosslinking a polymer composition via free radical formation using one or more free radical generating agents, wherein the crosslinking is effected using a compound of formula (I) and is effected by producing methane as a decomposition product of said crosslinking step in an amount of less than 300 ppm (weight), when determined according to a method as described below under "GC-analysis protocol". Preferably said crosslinking step is carried out without producing methane as a decomposition product of said crosslinking step. A preferable embodiment of said process for crosslinking a polymer composition by radical reaction, wherein said crosslinking is carried out using a free radical generating agent which results in CH₄ content of less than 300 ppm (weight), preferably of less than 200 ppm (weight), preferably of less than 100 ppm (weight), more preferably of from 0 to 50 ppm (weight) when measured as defined below under "GC-analysis protocol".

In demanding end application it is preferred that said crosslinking according to process (a) or (b) is carried out using a free radical generating agent which does not result in methane (CH₄) as a decomposition product thereof.

Preferably the crosslinking process is carried out under "crosslinking conditions" which means herein under free radical agent decomposing conditions. E.g. an elevated temperature is typically used for accelerating the decomposing of the radical and thus the crosslinking step. Moreover, "in the absence of CH₄ originating from said free radical generating agent" means that the free radical generating agent does not result in CH₄ as a decomposition product thereof during the crosslinking step.

The invention also provides a crosslinked polymer composition obtainable by the crosslinking process of the invention as defined above.

Said crosslinking process is further defined above under "1.Crosslinking of polymers".

### III. End Applications

### 1. Article

The new principle of the invention is highly feasible in wide variety of end applications of polymers.

### 1.1 Cable

In one preferable embodiment said article of the invention is a cable comprising a conductor surrounded with one or more layers, wherein at least one layer comprises said polymer composition of the invention.

The term "conductor" means herein above and below that the conductor comprises one or more wires. Moreover, the cable may comprise one or more such conductors. Preferably the conductor is an electrical conductor.

In one embodiment of the cable of the invention at least one layer is an insulation layer which comprises said polymer composition of the invention.

In another embodiment of the cable of the invention at least one layer is a semiconductive layer comprising said polymer composition of the invention. "Semiconductive layer" means herein that said layer comprises carbon black and has a volume resistivity of 100 000 Ω-cm or below when measured at 23°C or 90°C, or, when measured according to ISO 3915 using a plaque, has a volume resistivity of 100 Ω-cm or below at 23°C, or of 1000 Ω-cm or below at 90°C.

In further embodiment, the cable of the invention comprises a jacketing layer and optionally one or more layers selected from an insulation layer and semiconductive layer surrounded by said jacketing layer, wherein said jacketing layer comprises said polymer composition of the invention.

As one further embodiment of the cable of the invention, a low voltage cable is provided which comprises an insulation layer and optionally a jacketing layer, wherein said insulation layer comprises said polymer composition of the invention.

As a further embodiment of the cable of the invention, a power cable is provided which comprises at least an inner semiconductive layer, insulation layer and an outer semiconductive layer, in that order, optionally surrounded by a jacketing layer, wherein at least one of said layers, preferably at least inner semiconductive layer and insulation layer, comprises said polymer composition of the invention.

In the context of the present invention, a low voltage cable is a cable operating in voltages 1 kV or below. A power cable is defined to be a cable transferring energy operating at any voltage, typically operating at voltages higher than 1 kV. The voltage applied to the power cable can be alternating (AC), direct (DC), or transient (impulse). In a preferred embodiment, the power cable prepared according to the present invention is operating at voltages higher than 6 kV and are known i.a. as medium voltage (MV), high voltage (HV) and extra high voltage (EHV) power cables, which terms have well known meaning and indicate the operating level of such cable.

Said outer semiconductive layer of said power cable of the invention can be non-strippable, i.e. bonded and non-peelable, or strippable, i.e. non-bonded and peelable. Said terms have well known meanings in the wire and cable field.

### 2 Preparation process of an article

The present invention further provides a process for producing an article by using said polymer composition of the invention.

### 2.2. Preparation process of a cable

A preferable embodiment of the process for preparing an article of the invention is a process for producing a cable comprising steps of applying, preferably by (co)extrusion, one or more layers on a conductor, which layers comprise a polymer composition, wherein at least one layer comprises said polymer composition of the invention.

The term "(co)extrusion" means herein that in case of two or more layers, said layers can be extruded in separate steps, or at least two or all of said layers can be coextruded in a same extrusion step, as well known in the art.

In said process of the invention the components of a layer material are mixed in a separate mixer before introducing to the extruder for producing said layers or are added directly to an extruder and mixed therein before forming to a layer. Additives and further components can be added during the mixing step. The mixture in extruder is subjected to an elevated temperature, typically above the melting point of the polymer components and then (co)extruded on a conductor in a manner very well known in the field. E.g. conventional extruders and mixers may be used in the process of the invention.

The above described polymer powder, polymer pellets or melt of the invention, which comprise said polymer composition of the invention comprising Compound of the invention, can each equally be used in said process for preparing cables and they can be prepared prior their use in the cable preparation step or they can be prepared directly in a cable production line during a cable manufacturing process, as described above e.g. under "II. Polymer composition". Accordingly, 1) preformed powder or pellets of a polymer composition of the invention comprising Compound of the invention, may be subjected to the cable production line; or 2) said Compound of the invention may be added together with pellets or powder to a mixing step before forming the cable layer(s). Such mixing step can be a separate step in a separate mixing device arranged in the cable production line to precede the cable layer formation step, e.g. an extrusion step. Alternatively, Compound of the invention can be added during the layer formation step e.g. in an extruder, whereby it can be introduced to the extruder together with or after the addition of polymer powder or polymer pellets. The addition point in an extruder is not limited, whereby the Compound of the invention can be added at the inlet of the extruder or at a later feed point arranged along the extruder. Accordingly the addition of Compound of the invention may take place at the time the polymer material is in solid non-molten, partly molten or molten state, i.e. a melt mixture. The obtained molten mixture of a layer material is then (co)extruded on to a conductor to form a cable layer. In a preferred cable preparation process of the invention a low voltage cable or, more preferably, a power cable of the invention as defined above under 1.1. Cable is produced. The obtained cable can be further processed for the end use application.

Typically the cable of the invention is crosslinked after the formation of cable layers. The invention further provides a process for crosslinking a cable by radical reaction using one or more free radical generating agents, comprising step of: applying one or more layers comprising a polymer composition on a conductor, wherein at least one layer comprises one or more free radical generating agents, crosslinking by radical reaction said at least one layer comprising said free radical generating agent(s), and recovering the crosslinked cable in a conventional manner for further use; wherein in said process said crosslinking is effected using a compound of the invention and by producing methane as a decomposition product of said crosslinking step in an amount of less than 300 ppm (weight), when determined according to a method described below under "GC-analysis protocol", preferably said crosslinking step is carried out without producing methane as a decomposition product of said crosslinking step.

A further independent crosslinking process for crosslinking a cable by radical reaction using one or more free radical generating agents, comprising step of: applying one or more layers comprising a polymer composition on a conductor, wherein at least one layer comprises one or more free radical generating agents, crosslinking by radical reaction said at least one layer comprising said free radical generating agent(s), and recovering the crosslinked cable in a conventional manner for further use; wherein said crosslinking is carried out in the presence of Compound of the invention as a free radical generating agent. In another preferable embodiment this independent crosslinking process is dependent on the above crosslinking process wherein the features are defined by means of the decomposition products.

In above crosslinking process of the invention crosslinking conditions can vary depending i.a. on the used materials and cable size. The crosslinking of the invention is effected e.g. in a known manner preferably in an elevated temperature. Preferably the lowest temperature in a cable layer during the crosslinking step is above 140°C, more preferably above 150°C, such as 160-210°C. The crosslinking may be carried out in a liquid or gas medium, such as in an inert gas, such as N₂, atmosphere. The pressure during the crosslinking step of the invention is typically up to 20 bar, preferably up to 13 bar, such as 10-15 bar, in inert atmosphere. Said crosslinking step of the invention is also described above under "1. Crosslinking of polymers" and above under "II. Polymer composition".

A further preferable embodiment of the crosslinking process of the invention comprises a further step of cooling the crosslinked cable preferably under pressurized conditions in a cooling medium e.g. in gas or liquid, such as N₂, oil or water. The cooling is effected in a cooling zone, which may be optionally integrated with the preceding crosslinking zone, e.g. in a known vulcanization tube. As an example only, continuous catenary vulcanization (CCV) tube can be mentioned. The temperature at the layer closest to conductor is typically below 200°C, e.g. 160-190°C, at the beginning of the cooling zone/step. The pressure during the cooling step of the invention is typically kept above atmospheric pressure, e.g. up to 20 bar, preferably up to 13 bar, such as 10-12 bar. The cable is removed from the pressurized cooling step, when the temperature of the cable layers is clearly below the melting point of the polymer layer material thereof. Accordingly, the crosslinked cable of the invention may leave the pressurized cooling step of the invention e.g. when the temperature of the conductor of said cable is below 110°C depending on the layer polymer material, preferably between 70-90°C, at the exit of the pressurized cooling zone.

The crosslinking and cooling step is normally carried out under pressurized conditions to prevent the formation of voids due to volatile decomposition products of e.g. peroxides. In a preferable embodiment of the crosslinking process of the invention thus enables to remove the crosslinked and cooled cable from the pressurized cooling zone in a temperature higher than in the prior art, when measured from the conductor. Also preferably, the cooling may be effect at lower pressures compared to prior art.

Optionally, if desired, the crosslinked cable of the invention may be subjected to an additional non-pressurised cooling step after said pressurized cooling step, for further cooling of the cable.

The cable preparation process of the invention optionally comprises a further recovering step of the cable coming from the cooling step. Recovering may be effected by winding the cable on a cable drum in a known manner.

In a further embodiment of the process of the invention the cable obtained from the cooling step and optionally recovered, e.g. wound to a cable drum, may optionally be subjected, if needed in some applications, to a subsequent degassing step i.a. for removing or reducing any volatile decomposition products possibly resulting from said crosslinking step of the invention. In said degassing step the cable of the invention is preferably exposed either in ambient or elevated temperature for a period of time. As an example only, said degassing temperature may be e.g. 50-80°C, for a time period of one to four weeks. In one embodiment of the crosslinking process said degassing step may be shortened considerably or even avoided due to decreased level of said volatile by-products.

The cable of the invention produced by the above process of the invention may be further processed, e.g. protected with a protective layer, and/or optionally covered by a jacketing layer in a subsequent finishing step in a known manner and recovered for the end use thereof.

The invention thus provides also a crosslinked cable comprising crosslinked polymer composition as defined above, preferably a crosslinked low voltage cable or power cable, more preferably a crosslinked power cable, as defined above. Preferably said crosslinked cable is obtainable by any of the crosslinking process as defined above.

In one embodiment of a crosslinking process of the invention a crosslinked power cable is produced which is selected from a crosslinked MV cable, wherein the lowest degree of crosslinking in a cable layer(s) meets the requirements as specified in IEC 60502, or a crosslinked HV cable, wherein the lowest degree of crosslinking in a cable layer(s) meets the requirements as specified in IEC 60840, which specifications are well known in the W&C field.

The advantageous Compounds of the invention are preferable free radical generating agents which can be used for improving the quality of the products, e.g. in cable production processes. Due to the present invention the amount of voids in polymer products, such as cable layers can be reduced or even avoided, since less or no volatile decomposition products are formed from e.g. when Compound of the invention is used for modifying the polymer. Moreover, the invention also enables to improve the processability of a cable, i.a. in terms of safer and faster processing.

E.g. the crosslinking process of the invention can be faster and/or more economical, since both cooling and/or degassing steps may be carried out in a reduced time and/or in a less energy consuming manner, if desired.

### Determination method

### GC-Analysis protocol

In definitions of the Compounds, Polymer compositions, cables and preparation process and modification methods thereof as defined above and in claims below, the volatile, e.g. CH₄, content given in ppm (weight) or as "absent" is determined by gas chromatography (GC) from a sample which is modified, e.g. crosslinked according to following protocol:

The sample specimen of 1 g is taken from a modified, e.g. crosslinked composition. Or in case of a cable comprising a crosslinked layer(s) the sample specimen is taken from a layer material of a crosslinked and cooled cable sample that is taken at the exit of a crosslinking/cooling zone, such as at the exit of a vulcanisation tube, after pressurised cooling step in a manner known for a skilled person.

The collection of volatiles from said sample specimen (to a head space bottle, see below) is started within one hour after the modification step is stopped, or in case of a crosslinked and cooled cable, within one hour after the cable sample is taken at the exit of a crosslinking/cooling zone and sample specimen is prepared as described below:

A sample specimen of a thickness of 1 mm and of a weight of 1 g is cut in an axial direction from said cable sample from the middle distance (in radial direction) of the polymer layer(s) ring surrounding the conductor of said cable sample (i.e. at the distance of ½ radius of said cable layer ring). The obtained sample (specimen) is placed in a 120 ml head space bottle with an aluminium crimp cup with seal and heat treated at 60 ° C for 1.5 h for collecting any gaseous volatiles present in said sample. Then 0.3-0.5 ml of the gas captured in the sample bottle is injected into a gas chromatograph, wherein the presence and content of the volatiles, e.g. methane, which are desired to be measured in a known manner. Double samples are analysed and a "zero-sample" without free radical generating agent/modification is used as a reference. The instrument used herein was a Perkin Elmer 8500 with a Al₂O₃/KCl - column of 0,32mm x 50m, supplied by Perking Elmer.

### EXAMPLES

### Example 1

### Preparation of Di-(1-phenylcyclohexyl) peroxide

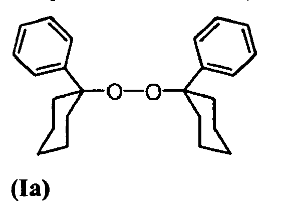

Di-(1-phenylcyclohexyl) peroxide (CAS: 21726-36-3) is prepared according to a procedure described in the literature, namely in article of Hey, D.H., Stirling, C.J.M., Williams, G.H, J. Chem. Soc., 1054-1058, 1957.

### Example 2

### Preparation of Di-(1-methyl-1-phenylundecyl) peroxide

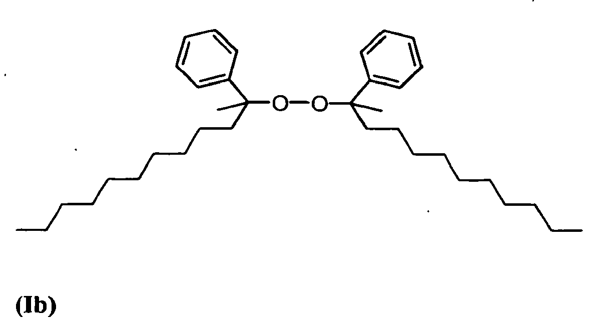

### A. 1-methyl-1-phenylundecyl alcohol

Acetophenone is dissolved in anhydrous diethylether, the temperature lowered to-78°C, followed by addition of decylmagnesium bromide. The reaction is performed at -78° for 20 min, stirred at RT for 1h, followed by quenching with H₂O. The organic layers are washed with H₂O, dried, and evaporated to yield the crude product. The product is purified using chromatography (hexane/DCM) on silica and used without further purification.

### B. Di-(1-methyl-1-phenylundecyl) peroxide

1-Methyl-1-phenylundecyl alcohol is dissolved in hexane at 4°C and to this solution 70% H₂SO₄ is added followed by addition of H₂O₂ dissolved in H₂SO₄ and stirred for 4h. The reaction mixture is then separated and the organic phase washed with H₂O, dried, and evaporated to yield the crude product. This crude product is recrystallized to give di-(1-methyl-1-phenylundecyl) peroxide.

### Example 3

### Preparation of Di-(1-decyl-1-phenylundecyl) peroxide

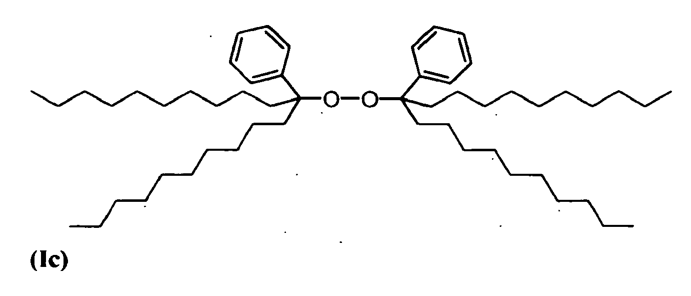

### A. 1-decyl-1-phenylundecyl alcohol

Ethyl benzoate is dissolved in anhydrous diethylether, the temperature lowered to-78°C, followed by addition of decylmagnesium bromide. The reaction is performed at -78° for 20 min, stirred at RT for 1h, followed by quenching with H₂O. The organic layers are washed with H₂O, dried, and evaporated to yield the crude product. The product is purified using chromatography (hexane/DCM) on silica and used without further purification.

### B. Di-(1-decyl-1-phenylundecyl) peroxide

1-decyl-1-phenylundecyl alcohol is dissolved in hexane at 4°C and to this solution 70% H₂SO₄ is added followed by addition of H₂O₂ dissolved in H₂SO₄ and stirred for 4h. The reaction mixture is then separated and the organic phase washed with H₂O, dried, and evaporated to yield the crude product. This crude product is recrystallized to give di-(1-decyl-1-phenylundecyl) peroxide.

### Preparation example of the crosslinked cable of the invention:

A power cable comprising an inner semiconductive layer, an insulation layer and an outer semiconductive layer for experimental testing is prepared by coextruding on a conductor said layers in given order using a conventional extruder line and conventional extrusion conditions.
The layer materials are conventional polymer grades and each layer comprises a peroxide compound of the invention as a crosslinking agent.
The semiconductive material used in the cable, both as inner and outer semicon, is a poly(ethylene-co-butylacrylate) polymer (with a butylacrylate content of 17wt%) containing 40 wt% of a furnace black. The composition is stabilised with an antioxidant of the polyquinoline type and contains 1 wt% of the peroxide of the invention as a crosslinking agent.

The middle insulation layer is formed of low density polyethylene LDPE (MFR₂=2 g/10 min) containing 2 wt-% of the peroxide of the invention and 0.2 wt-% of 4,4'-thiobis(2-tert.-butyl-5-methylphenol).

The obtained cable is immediately after extrusion subjected to a conventional vulcanisation tube and crosslinked in a known manner using well known crosslinking conditions. After crosslinking the cable is then cooled in cooling zone of said vulcanisation tube. The crosslinked and cooled layer is wound to a cable drum and then the methane content is determined.

## Claims

1. A compound which is an organic peroxide of formula (I) 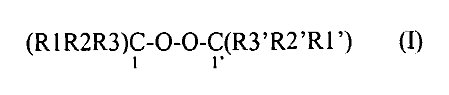 wherein
- R1 and R1' are each independently substituted or unsubstituted saturated or partially unsaturated hydrocarbyl; or substituted or unsubstituted aromatic hydrocarbyl;
- wherein each of said substituted or unsubstituted saturated or partially unsaturated hydrocarbyl or aromatic hydrocarbyl optionally comprises one or more heteroatoms;
- wherein said substituted or unsubstituted saturated or partially unsaturated hydrocarbyl include straight or branched chain saturated or partially unsaturated hydrocarbyls; straight or branched chain saturated or partially unsaturated hydrocarbyls which bear saturated or partially unsaturated cyclic hydrocarbyl; and saturated or partially unsaturated cyclic hydrocarbyls;
- wherein each of said aromatic hydrocarbyl and said saturated or partially unsaturated cyclic hydrocarbyl is independently a monocyclic or multicyclic ring system; and
- wherein said substituted saturated or partially unsaturated hydrocarbyl or substituted aromatic hydrocarbyl comprises independently 1 to 4 substituents selected from a functional group, saturated or partially unsaturated hydrocarbyl optionally bearing a functional group; or aromatic hydrocarbyl optionally bearing a functional group;
- R2, R2', R3 and R3' are each independently H, substituted or unsubstituted saturated or partially unsaturated hydrocarbyl; or substituted or unsubstituted aromatic hydrocarbyl;
- wherein each of said substituted or unsubstituted saturated or partially unsaturated hydrocarbyl or aromatic hydrocarbyl optionally comprises one or more heteroatoms;
- wherein said substituted or unsubstituted saturated or partially unsaturated hydrocarbyl include straight or branched chain saturated or partially unsaturated hydrocarbyls; straight or branched chain saturated or partially unsaturated hydrocarbyls which bear saturated or partially unsaturated cyclic hydrocarbyl; and saturated or partially unsaturated cyclic hydrocarbyls;
- wherein each of said aromatic hydrocarbyl and said saturated or partially unsaturated cyclic hydrocarbyl is independently a monocyclic or multicyclic ring system; and
- wherein said substituted saturated or partially unsaturated hydrocarbyl or substituted aromatic hydrocarbyl comprises independently 1 to 4 substituents selected from a functional group, saturated or partially unsaturated hydrocarbyl optionally bearing a functional group; or aromatic hydrocarbyl optionally bearing a functional group; or
- R2 and R3 together with the carbon atom (C1) to which they are attached form unsubstituted or substituted saturated or partially unsaturated carbocyclic ring moiety of 3 to 14 C-atoms; unsubstituted or substituted saturated or partially unsaturated heteroring moiety of 3 to 14 ring atoms comprising 1 to 6 heteroatoms selected from O, N, P, S or Si; or unsubstituted or substituted aromatic ring moiety of 3 to 14 C-atoms optionally comprising 1 to 4 heteroatoms;
- wherein said carbocyclic ring, heteroring or aromatic ring system is optionally fused with another ring system having 4 to 14 ring atoms; and
- wherein said substituted carbocyclic ring, heteroring or aromatic ring system comprises 1 to 4 substituents selected independently from a functional group or saturated or partially unsaturated hydrocarbyl optionally bearing a functional group or aromatic hydrocarbyl optionally bearing a functional group; or
- R2' and R3' together with the carbon atom (C1') to which they are attached form unsubstituted or substituted saturated or partially unsaturated carbocyclic ring moiety of 3 to 14 C-atoms; unsubstituted or substituted saturated or partially unsaturated heteroring moiety of 3 to 14 ring atoms comprising 1 to 6 heteroatoms selected from O, N, P, S or Si; or unsubstituted or substituted aromatic ring moiety of 3 to 14 C-atoms optionally comprising 1 to 4 heteroatoms;
- wherein said carbocyclic ring, heteroring or aromatic ring system is optionally fused with another ring system having 4 to 14 ring atoms; and
- wherein said substituted carbocyclic ring, heteroring or aromatic ring system comprises 1 to 4 substituents selected independently from a functional group or a saturated or partially unsaturated hydrocarbyl optionally bearing a functional group or aromatic hydrocarbyl optionally bearing a functional group; or
- R2 and R2' form together a bivalent substituted or unsubstituted saturated or partly unsaturated hydrocarbyl optionally containing 1 to 4 heteroatoms, wherein R2 is linked to C1 and R2' to C1', respectively, forming together with -C1-O-O-C1'-substituted or unsubstituted saturated or partially unsaturated carbocyclic ring moiety of 3 to 14 C-atoms comprising optionally, in addition to said at least two O atoms, 1 to 4 further heteroatoms; wherein said carbocyclic ring or heteroring system is optionally fused with another ring system having 4-14 ring atoms;
and functional derivatives thereof;
- with a proviso that at least two of R1, R2 and R3, and at least two of R1', R2' and R3', respectively, are other than H or methyl.

2. A compound of formula (I) as defined in claim 1, wherein R2 and R3 together with carbon atom (C1) to which they are attached form an optionally substituted carbocyclic ring moiety of 3 to 12 ring C-atoms or an optionally substituted heteroring moiety of 3 to 12 ring atoms containing 1 to 6, preferably 1 to 4, heteroatoms selected from O, N, P, S or Si, and wherein said carbocyclic or heterocyclic ring system is optionally fused with another ring system having 4 to 14 ring atoms, preferably R2 and R3 together with carbon atom (C1) form a (C3-C12)carbocyclic ring moiety; preferably an optionally substituted, saturated or partially unsaturated mono- or bicyclic (C4-C14)carbocyclic ring, preferably unsubstituted saturated monocyclic (C5-C8)carbocyclic ring.

3. A compound of formula (1) as defined in claim 1, wherein R2' and R3' together with carbon atom (C1') to which they are attached form an optionally substituted carbocyclic ring moiety of 3 to 12 ring C-atoms or an optionally substituted heteroring moiety of 3 to 12 ring atoms containing 1 to 6, preferably 1 to 4, heteroatoms selected from O, N, P, S or Si, and wherein said carbocyclic or heterocyclic ring system is optionally fused with another ring system having 4 to 14 ring atoms, preferably R2 and R3 together with carbon atom (C1') form a (C3-C 12)carbocyclic ring moiety; preferably an optionally substituted, saturated or partially unsaturated mono- or bicyclic (C4-C 14)carbocyclic ring, preferably unsubstituted saturated monocyclic (C5-C8)carbocyclic ring.

4. A compound of formula (I) as defined in any of the preceding claims 1-3, wherein R2 and R3 together with the carbon atom (C1) to which they are attached form a ring system as defined in claim 2 and R2' and R3' together with the carbon atom (C1') to which they are attached form a ring system as defined in claim 3, and wherein the ring system formed by R2' and R3' together with the carbon atom (C1') to which they are attached is same as the ring system formed by R2 and R3 together with the carbon atom (C1) to which they are attached.

5. A compound of formula (I) as defined in claim 1, wherein R1, R2, R3, R1', R2' and R3' each independently is optionally substituted mono- or multicyclic (C5-C 14)aryl; optionally substituted mono- or multicyclic (C5-C14)heteroaryl; optionally substituted mono- or multicyclic (C4-14)cycloalkyl; optionally substituted mono- or multicyclic (C4-C 14)heterocyclyl; optionally substituted straight or branched chain (C1-C50)alkyl, preferably straight chain (C1-C30)alkyl; optionally substituted straight or branched chain (C1-C50)alkenyl or optionally substituted straight or branched chain (C1-C50)alkynyl, preferably straight chain (C1-C30)alkenyl or straight chain (C1-C30)alkynyl; optionally substituted straight or branched chain (C1-C50)heteroalkyl comprising 1 to 4 heteroatoms selected from O, N, P, S or Si; preferably R2, R2', R3 and R3' are independently selected from unsubstituted straight chain (C1-C50)alkyl, preferably (C1-C30)alkyl, more preferably (C1-C20)alkyl.

6. A compound of formula (I) as defined as defined in claim 1-5, wherein R2 and R2' each represents same radical and, respectively, R3 and R3' each represents same radical.

7. A compound of formula (I) as defined in claim 1, 5 or 6, selected from wherein
- R2 and R2' are same and each represents methyl; or
- R2 and R2' are same and each represents (C6-C30)alkyl.

8. A compound of formula (I) as defined in claim 1 or 5-7, wherein R3 and R3' are same and each represents (C6-C30)alkyl.

9. A compound of formula (I) as defined in any of the preceding claims 1-8, selected from wherein
- R1 and R1' are same or different, preferably same, and each represents optionally substituted, saturated or partially unsaturated cyclic hydrocarbyl of 5 to 14 ring atoms optionally containing 1 to 4 heteroring atoms selected from N, O, P, S or Si; or optionally substituted mono- or multicyclic (C5-C14)aryl, preferably unsubstituted monocyclic (C5-C7)aryl; or
- R1 and R2' are same or different, preferably same, and each represents optionally substituted branched or straight chain, preferably unsubstituted straight chain, (C6-C30)alkyl or methyl.

10. A compound of formula (I) as defined in any of the preceding claims 1-9, wherein when more than one of R1, R2, R3 is an alkyl as defined in any of previous claims 1 or 5-9 and one of R1, R2, R3 as said alkyl is methyl, preferably (C1-C3)alkyl, then the rest of R1, R2 and R3 as said alkyl represent(s) independently (C6-C20)alkyl or, respectively, when more than one of R1', R2', R3' is an alkyl as defined in any of previous claims 1 or 5-9 and one of R1', R2', R3'as said alkyl is methyl, then the rest of R1', R2' and R3' as said alkyl represent(s) independently (C6-C20)alkyl.

11. A compound of formula (I) as claimed in claim 1 which is selected from any of
- Di-(1-methyl-1-phenylundecyl) peroxide (formula Ib), or
- Di-(1-decyl-1-phenylundecyl) peroxide (formula Ic).

12. The use of a compound as defined in any of claims 1-11 as a free radical generating agent for modifying polymers.

13. The use of a compound as defined in claim 12, selected from the following uses for:
(1) crosslinking of polymers via radical reaction;
(2) grafting polymers with compounds, preferably unsaturated compounds, via radical reaction; or
(3) visbreaking a polymer to modify melt flow rate (MFR) of said polymer via radical reaction ; preferably for (1) crosslinking of polymers.

14. The use of a compound as defined in claim 12 or 13 for modifying polymers in wire and cable applications.

15. A polymer composition comprising a compound as defined in any of claims 1-11.

16. A polymer composition as defined in claim 15 which is in a form of (1) polymer powder, (2) polymer pellets or (3) a mixture of a polymer melt,
whereby said (1) polymer powder or, preferably, (2) polymer pellets are optionally contained in a container.

17. A process for crosslinking a polymer composition by a radical reaction using one or more free radical generating agents **characterized in that** the crosslinking is effected using a free radical generating agent which is a compound as defined in any of claims 1-11.

18. The process as defined in claim 17 **characterized in that** the crosslinking is effected by producing methane as a decomposition product of said crosslinking step in an amount of less than 300 ppm (weight), when determined from the produced crosslinked polymer sample according to a method as described in the description under "GC-analysis protocol", preferably said crosslinking step is carried out without producing methane as a decomposition product of said crosslinking step.

19. A crosslinked composition obtainable by a crosslinking process of claim 17 comprising CH4 in an amount of less than 300 ppm (weight), preferably of less than 200 ppm (weight), suitably of less than 100 ppm (weight), preferably in an amount of 0 to 50 ppm (weight), as a decomposition product of said crosslinking step, when determined from the crosslinked composition sample according to a method as described in the description under "GC-analysis protocol",

20. A crosslinkable cable which comprises a conductor which is surrounded by one or more layers comprising a polymer composition, **characterized in that** at least one layer comprises a polymer composition as defined in claim 15 or 16.

21. A crosslinkable cable as defined in claim 20 which comprises at least an insulation layer which comprises a polymer composition as defined in claim 15 or 16.

22. A crosslinkable cable as defined in claim 20 or 21 which comprises at least one semiconductive layer comprising a polymer composition as defined in claim 15 or 16.

23. A crosslinkable cable as defined in claim 20, 21 or 22 which comprises a jacketing layer and optionally one or more layers selected from an insulation layer and semiconductive layer surrounded by said jacketing layer, wherein said jacketing layer comprises a polymer composition as defined in claim 15 or 16.

24. A crosslinkable cable as defined in any of the preceding claims 20-23 which is selected from any of the following cables:
- a low voltage cable comprising a conductor surrounded by an insulation layer and optionally a jacketing layer, wherein said insulation layer comprises a polymer composition as defined in claim 15 or 16; or
- a power cable comprising an electrical conductor surrounded by one or more layers comprising at least an inner semiconductive layer, insulation layer and an outer semiconductive layer, in that order, and optionally surrounded by a jacketing layer, wherein at least one of said layers comprises, preferably at least inner semiconductive layer and insulation layer, a polymer composition as defined in claim 15 or 16.

25. A process for producing a crosslinkable cable comprising step of applying one or more layers comprising a polymer composition on a conductor, **characterized in that** in said at least one layer a polymer composition as defined in claim 15 or 16 is used.

26. The process as defined in claim 25 for preparing a crosslinkable cable as defined in any of the preceding claims 20-24.

27. A crosslinkable cable obtainable by a process as defined in claim 25 or claim 26, preferably a crosslinkable low voltage cable or a crosslinkable power cable.

28. A process for crosslinking a cable by radical reaction, comprising:
- applying one or more layers comprising a polymer composition on a conductor, wherein at least one layer comprises one or more free radical generating agents, and
- crosslinking by radical reaction said at least one layer comprising said free radical generating agent(s),**characterized in that** said crosslinking is carried out using a process as defined in claim 17 or 18.

29. The process of claim 28 for crosslinking a cable which is prepared according to claim 25 or claim 26.

30. The process of any of the preceding claims 28-29, wherein the crosslinked cable thus obtained is subjected to a further cooling step, wherein said crosslinked cable is cooled under pressurized conditions,
and, optionally, after said cooling step the crosslinked and cooled cable is subjected to one or more additional steps selected from:
- a non-pressurized cooling step, wherein the crosslinked and cooled cable is further cooled in a cooling medium,
- a recovering step, wherein the crosslinked cable is collected after the cooling step, preferably wound to a cable drum,
- a degassing step, wherein the content of volatile decomposition products(s) is reduced or removed, optionally at ambient or in elevated temperature, from said crosslinked cable obtained from said cooling and optional recovery step, and/or
- a finishing step, wherein the obtained crosslinked cable is finished in a conventional manner for further use.

31. A crosslinked cable obtainable by a process as defined in any of the preceding claims 28-30, preferably a crosslinked low voltage cable or a crosslinked power cable.

32. A use of a compound of formula (I) selected from one or more of:
- Di-(1-phenylcyclohexyl) peroxide (formula Ia),
- Di-(1-methyl-1-phenylundecyl) peroxide (formula Ib), or
- Di-(1-decyl-1-phenylundecyl) peroxide (formula Ic),
as a free radical generating agent in a product or a process as defined in any of the preceding claims 14-31, preferably as a crosslinking agent.

## Patentansprüche

1. Eine Verbindung, welche ein organisches Peroxid der Formel (I) ist 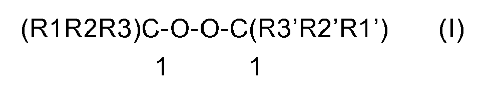 worin
- R1 und R1' jedes unabhängig voneinander ein substituierter oder unsubstituierter gesättigter oder teilweise ungesättigter Kohlenwasserstoff ist; oder ein substituierter oder unsubstituierter aromatischer Kohlenwasserstoff;
- worin jeder der substituierten oder unsubstituierten gesättigten oder teilweise ungesättigten Kohlenwasserstoffe oder aromatischen Kohlenwasserstoffe optional ein oder mehrere Heteroatome umfasst;
- worin der substituierte oder unsubstituierte gesättigte oder teilweise ungesättigte Kohlenwasserstoff geradkettige oder verzweigtkettige, gesättigte oder teilweise ungesättigte Kohlenwasserstoffe umfasst; geradkettige oder verzweigtkettige gesättigte oder teilweise ungesättigte Kohlenwasserstoffe, die gesättigten oder teilweise ungesättigten zyklischen Kohlenwasserstoff tragen; und gesättigte oder teilweise ungesättigte zyklische Kohlenwasserstoffe;
- worin jeder der aromatischen Kohlenwasserstoffe und der gesättigten oder teilweise ungesättigten zyklischen Kohlenwasserstoffe unabhängig voneinander ein monozyklisches oder multizyklisches Ringsystem ist; und
- worin der substituierte gesättigte oder teilweise ungesättigte Kohlenwasserstoff oder substituierte aromatische Kohlenwasserstoff unabhängig voneinander 1 bis 4 Substituenten umfasst, ausgewählt aus einer funktionellen Gruppe, einem gesättigten oder teilweise ungesättigten Kohlenwasserstoff, welcher optional eine funktionelle Gruppe trägt; oder einem aromatischen Kohlenwasserstoff, welcher optional eine funktionelle Gruppe trägt;
- R2, R2', R3 und R3' jeweils unabhängig voneinander H, substituierter oder unsubstituierter gesättigter oder teilweise ungesättigter Kohlenwasserstoff sind; oder substituierter oder unsubstituierter aromatischer Kohlenwasserstoff;
- worin jeder der substituierten oder unsubstituierten gesättigten oder teilweise ungesättigten oder aromatischen Kohlenwasserstoffe optional ein oder mehrere Heteroatome umfasst;
- worin der substituierte oder unsubstituierte gesättigte oder teilweise ungesättigte Kohlenwasserstoff geradkettige oder verzweigtkettige, gesättigte oder teilweise ungesättigte Kohlenwasserstoffe beinhaltet; geradkettige oder verzweigtkettige, gesättigte oder teilweise ungesättigte Kohlenwasserstoffe, die gesättigten oder teilweise ungesättigten zyklischen Kohlenwasserstoff tragen; und gesättigte oder teilweise ungesättigte zyklische Kohlenwasserstoffe;
- worin jeder der aromatischen Kohlenwasserstoffe und der gesättigten oder teilweise ungesättigten zyklischen Kohlenwasserstoffe unabhängig voneinander ein monozyklisches oder multizyklisches Ringsystem ist; und
- worin der substituierte gesättigte oder teilweise ungesättigte Kohlenwasserstoff oder der substituierte aromatische Kohlenwasserstoff unabhängig voneinander 1 bis 4 Substituenten umfasst, ausgewählt aus einer funktionellen Gruppe, einem gesättigten oder teilweise ungesättigten Kohlenwasserstoff, welcher optional eine funktionelle Gruppe trägt; oder einem aromatische Kohlenwasserstoff, welcher optional eine funktionelle Gruppe trägt; oder
- R2 und R3 zusammen mit dem Kohlenstoffatom (C1), an das sie gebunden sind, eine unsubstituierte oder substituierte gesättigte oder teilweise ungesättigte karbozyklische Ringeinheit mit 3 bis 14 C-Atomen bilden; einen unsubstituierte oder substituierte gesättigte oder teilweise ungesättigte Heteroringeinheit mit 3 bis 14 Ringatomen umfassend 1 bis 6 Heteroatome ausgewählt aus O, N P, S oder Si; oder eine unsubstituierte oder substituierte aromatische Ringeinheit umfassend 3 bis 14 C-Atome, optional umfassend 1 bis 4 Heteroatome;
- worin das karbozyklischen Ring-, Heteroring- oder aromatische Ringsystem optional mit einem anderen Ringsystem, das 4 bis 14 Ringatome hat, kondensiert ist; und
- worin das substituierte karbozyklische Ring-, Heteroring- oder aromatische Ringsystem optional 1 bis 4 Substituenten umfasst, unabhängig voneinander ausgewählt aus einer funktionellen Gruppe oder gesättigtem oder teilweise ungesättigtem Kohlenwasserstoff, welcher optional eine funktionelle Gruppe trägt, oder aromatischem Kohlenwasserstoff, welcher optional eine funktionelle Gruppe trägt; oder
- R2'und R3' zusammen mit dem Kohlenstoffatom (C1'), an das sie gebunden sind, eine unsubstituierte oder substituierte gesättigte oder teilweise ungesättigte karbozyklische Ringeinheit mit 3 bis 14 C-Atomen bilden; eine unsubstituierte oder substituierte gesättigte oder teilweise ungesättigte Heteroringeinheit mit 3 bis 14 Ringatomen umfassend 1 bis 6 Heteroatome, ausgewählt aus O, N, P, S oder Si; oder eine unsubstituierte oder substituierte aromatische Ringeinheit mit 3 bis 14 C-Atomen, optional umfassend 1 bis 4 Heteroatome;
- worin das karbozyklische Ring-, Heteroring- oder aromatische Ringsystem optional mit einem weiteren Ringsystem, welcher 4 bis 14 Ringatome hat, kondensiert ist; und
- worin das substituierte karbozyklische Ring-, Heteroring- oder aromatische Ringsystem 1 bis 4 Substituenten umfasst, unabhängig voneinander ausgewählt aus einer funktionellen Gruppe oder einem gesättigten oder teilweise ungesättigten Kohlenwasserstoff, welcher optional eine funktionelle Gruppe trägt, oder aromatischen Kohlenwasserstoff, welcher optional eine funktionelle Gruppe trägt; oder
- R2 und R2' zusammen einen zweiwertigen substituierten oder unsubstituierten gesättigten oder teilweise ungesättigten Kohlenwasserstoff bilden, welcher optional 1 bis 4 Heteroatomen beinhaltet, worin R2 mit C1 und R2' mit C1' verbunden ist, beziehungsweise welche zusammen mit -C1-O-O-C1'- eine substituierte oder unsubstituierte gesättigte oder teilweise ungesättigte karbozyklische Ringeinheit mit 3 bis 14 C-Atomen bildet, die optional, zusätzlich zu den zumindest zwei O-Atomen, 1 bis 4 weitere Heteroatome umfassen; worin das karbozyklische Ring- oder Heteroringsystem optional mit einem anderen Ringsystem mit 4-14 Ringatomen kondensiert ist; und funktionelle Derivate davon;
- mit der Maßgabe, dass mindestens zwei von R1, R2 und R3 beziehungsweise mindestens zwei von R1', R2' und R3' etwas anderes als H oder Methyl sind.

2. Eine Verbindung der Formel (I) gemäß Anspruch 1, worin R2 und R3 zusammen mit dem Kohlenstoffatom (C1), an das sie gebunden sind, eine optional substituierte, karbozyklischen Ringeinheit mit 3 bis 12 Ring-C-Atomen oder eine optional substituierte Heteroringeinheit mit 3 bis 12 Ringatomen bilden, die 1 bis 6, vorzugsweise 1 bis 4 Heteroatome beinhalten, ausgewählt aus O, N, P, S oder Si, und worin das karbozyklische oder heterozyklische Ringsystem optional mit einem weiteren Ringsystem mit 4 bis 14 Ringatomen kondensiert ist, vorzugsweise bilden R2 und R3 zusammen mit dem Kohlenstoffatom (C1) eine (C3-C12)-karbozyklische Ringeinheit; vorzugsweise einen optional substituierten, gesättigten oder teilweise ungesättigten mono- oder bizyklischen (C4-C14)-karbozyklischen Ring, vorzugsweise einen unsubstituierten gesättigten monozyklischen (C5-C8)-karbozyklischen Ring.

3. Eine Verbindung der Formel (I) gemäß Anspruch 1, worin R2' und R3' zusammen mit dem Kohlenstoffatom (C1'), an das sie gebunden sind, eine optional substituierte karbozyklische Ringeinheit mit 3 bis 12 Ring-C-Atomen oder eine optional substituierte Heteroringeinheit mit 3 bis 12 Ringatomen bilden, welche 1 bis 6, vorzugsweise 1 bis 4 Heteroatome beinhalten, ausgewählt aus O, N, P, S oder Si, und worin das karbozyklische oder heterozyklische Ringsystem optional mit einem weiteren Ringsystem mit 4 bis 14 Ringatomen kondensiert ist, vorzugsweise bilden R2 und R3 zusammen mit dem Kohlenstoffatom (C1') eine (C3-C12)-karbozyklische Ringeinheit; vorzugsweise eine optional substituierten, gesättigten oder teilweise ungesättigten mono- oder bizyklischen (C4-C14)-karbozyklischen Ring, vorzugsweise einen unsubstituierten gesättigten monozyklischen (C5-C8)-karbozyklischen Ring.

4. Eine Verbindung der Formel (I) gemäß einem der vorangehenden Ansprüche 1-3, worin R2 und R3 zusammen mit dem Kohlenstoffatom (C1), an das sie gebunden sind, ein Ringsystem gemäß Anspruch 2 bilden und R2' und R3' zusammen mit dem Kohlenstoffatom (C1'), an das sie gebunden sind, ein Ringsystem gemäß Anspruch 3 bilden und worin das Ringsystem, welches von R2' und R3' zusammen mit dem Kohlenstoffatom (C1') gebildet wird, an das sie gebunden sind, dasselbe ist wie das Ringsystem, welches von R2 und R3 zusammen mit dem Kohlenstoffatom (C1), an das sie gebunden sind, gebildet wird.

5. Eine Verbindung der Formel (I) gemäß Anspruch 1, worin R1, R2, R3, R1', R2' und R3' jeweils unabhängig voneinander optional substituiertes mono- oder multizyklisches (C5-C14)-Aryl ist; optional substituiertes mono- oder multizyklisches (C5-C14)-Heteroaryl; optional substituiertes mono- oder multizyklisches (C4-C14)-Cycloalkyl; optional substituiertes mono- oder multizyklisches (C4-C14)-Heterocyclyl; optional substituiertes geradkettiges oder verzweigtkettiges (C1-C50)-Alkyl, vorzugsweise geradkettiges (C1-C30)-Alkyl; optional substituiertes geradkettiges oder verzweigtkettiges (C1-C50)-Alkenyl oder optional substituiertes geradkettiges oder verzweigtkettiges (C1-C50)-Alkinyl, vorzugsweise geradkettiges (C1-C30)-Alkenyl oder geradkettiges (C1-C30)-Alkinyl; optional substituiertes gerad- oder verzweigtkettiges (C1-C50)-Heteroalkyl, welches 1 bis 4 Heteroatome umfasst, ausgewählt aus O, N, P, S oder Si, wobei R2, R2', R3 und R3' vorzugsweise unabhängig voneinander ausgewählt sind aus einem unsubstituierten geradkettigen (C1-C50)-Alkyl, vorzugsweise (C1-C30)-Alkyl, besonders bevorzugt (C1-C20)-Alkyl.

6. Eine Verbindung der Formel (I) gemäß Anspruch 1-5, sind R2 und R2' jeweils dasselbe Radikal beziehungsweise R3 und R3' jeweils dasselbe Radikal repräsentieren.

7. Eine Verbindung der Formel (I) gemäß Anspruch 1, 5 oder 6, ausgewählt aus, worin
- R2 und R2' gleich sind und jeweils Methyl repräsentieren; oder
- R2 und R2' gleich sind und jeweils ein (C6-C30)-Alkyl repräsentieren.

8. Eine Verbindung der Formel (I) gemäß Anspruch 1 oder 5-7, worin R3 und R3' gleich sind und jeweils ein (C6-C30)-Alkyl repräsentieren.

9. Eine Verbindung der Formel (I) gemäß einem der vorangehenden Ansprüche 1-8, ausgewählt aus, worin
- R1 und R1' gleich oder verschieden sind, vorzugsweise gleich, und jeweils optional substituierten, gesättigten oder teilweise ungesättigten zyklischen Kohlenwasserstoff mit 5 bis 14 Ringatomen repräsentieren, die optional 1 bis 4 Heteroatome beinhalten, ausgewählt aus N, O, P, S oder Si; oder optional substituiertes mono- oder multizyklische (C5-C14)-Aryl, vorzugsweise unsubstituierte monozyklische (C5-C7)-Aryl; oder
- R1 und R1'gleich oder verschieden sind, bevorzugt gleich, und jeweils optional substituiertes verzweigtkettiges oder geradkettiges, vorzugsweise unsubstituiertes geradkettiges (C6-C30)-Alkyl oder Methyl repräsentiert.

10. Eine Verbindung der Formel (I) gemäß einem der vorangehenden Ansprüche 1-9, worin, wenn mehr als eines von R1, R2, R3 ein Alkyl gemäß einem der vorangehenden Ansprüche 1 oder 5-9 und eines von R1, R2, R3 wie dieses Alkyl Methyl ist, vorzugsweise ein (C1-C3)-Alkyl, dann repräsentiert(en) der Rest von R1, R2 und R3 wie dieses Alkyl unabhängig voneinander (C6-C20)-Alkyl, oder beziehungsweise, wenn mehr als eines von R1 R2', R3' ein Alkyl gemäß einem der vorangehenden Ansprüche 1 oder 5-9 ist und eines von R1', R2', R3' wie dieses Alkyl Methyl ist, dann repräsentiert(en) der Rest von R1', R2' und R3' wie dieses Alkyl unabhängig voneinander ein (C6-C20)-Alkyl.

11. Eine Verbindung der Formel (I) gemäß Anspruch 1, die ausgewählt ist aus
- Di-(1-methyl-1-phenylundecyl)peroxid (Formel Ib), oder
- Di-(1-decyl-1-phenylundecyl)peroxid (Formel Ic).

12. Die Verwendung einer Verbindung gemäß einem der Ansprüche 1-11 als Radikalerzeugungsmittel zum Modifizieren von Polymeren.

13. Die Verwendung einer Verbindung gemäß Anspruch 12, ausgewählt aus den folgenden Verwendungen:
(1) Vernetzung von Polymeren durch radikalische Reaktion;
(2) Pfropfen von Polymeren mit Verbindungen, vorzugsweise ungesättigte Verbindungen, über eine Radikalreaktion; oder
(3) Visbreaking eines Polymers, um die Schmelzflussrate (MFR) des Polymers über eine Radikalreaktion zu modifizieren, vorzugsweise zur (1) Vernetzung von Polymeren.

14. Die Verwendung einer Verbindung gemäß den Ansprüchen 12 oder 13 zum Modifizieren von Polymeren in Draht- und Kabelanwendungen.

15. Polymerzusammensetzung, umfassend eine Verbindung gemäß einem der Ansprüche 1-11.

16. Polymerzusammensetzung gemäß Anspruch 15, die in Form von (1) Polymerpulver, (2) Polymergranulat oder (3) einem Gemisch aus einer Polymerschmelze vorliegt, wobei das (1) Polymerpulver oder vorzugsweise (2) Polymergranulat optional in einem Behälter enthalten sind.

17. Verfahren zum Vernetzrn einer Polymerzusammensetzung durch eine radikalische Reaktion unter Verwendung eines oder mehrerer Radikalbildner, **dadurch gekennzeichnet, dass** das Vernetzen unter Verwendung eines freie Radikale erzeugenden Mittels, das eine Verbindung gemäß einem der Ansprüche 1-11 ist, durchgeführt wird.

18. Das Verfahren gemäß Anspruch 17, **dadurch gekennzeichnet, dass** das Vernetzen bei Erzeugung von Methan als Zersetzungsprodukt des Vernetzungsschrittes in einer Menge von weniger als 300 ppm (Gewicht) durchgeführt wird, wenn es an der hergestellten vernetzten Polymerprobe nach einem Verfahren wie in der Beschreibung unter "GC-Analyseprotokoll" beschrieben bestimmt wird, wobei der Vernetzungsschritt vorzugsweise erfolgt, ohne dass Methan als Zersetzungsprodukt des Vernetzungsschritt produziert wird.

19. Vernetzte Zusammensetzung erhältlich durch ein Vernetzungsverfahren gemäß Anspruch 17, umfassend CH₄ in einer Menge von weniger als 300 ppm (Gewicht), vorzugsweise von weniger als 200 ppm (Gewicht), geeigneterweise von weniger als 100 ppm (Gewicht), vorzugsweise in einer Menge von 0 bis 50 ppm (Gewicht) als Zersetzungsprodukt des Vernetzungsschrittes, wenn es an der vernetzten Zusammensetzungsprobe nach einem Verfahren, wie in der Beschreibung unter "GC-Analyseprotokoll" beschrieben, bestimmt wird.

20. Vernetzbares Kabel, das einen Leiter umfasst, der von einer oder mehreren Schichten umfassend eine Polymerzusammensetzung umgeben ist, **dadurch gekennzeichnet, dass** die mindestens eine Schicht eine Polymerzusammensetzung gemäß Anspruch 15 oder 16 umfasst.

21. Vernetzbares Kabel gemäß Anspruch 20, das mindestens eine Isolationsschicht umfasst, die eine Polymerzusammensetzung gemäß Anspruch 15 oder 16 umfasst.

22. Vernetzbares Kabel gemäß Anspruch 20 oder 21, das mindestens eine halbleitende Schicht umfasst, die eine Polymerzusammensetzung gemäß Anspruch 15 oder 16 umfasst.

23. Vernetzbares Kabel, gemäß Anspruch 20, 21 oder 22, das eine Mantelschicht und optional eine oder mehrere Schichten, ausgewählt aus einer Isolationsschicht und einer halbleitenden Schicht, umfasst, die von der Mantelschicht umgeben sind, worin die Mantelschicht eine Polymerzusammensetzung gemäß Anspruch 15 oder 16 umfasst.

24. Vernetzbares Kabel gemäß einem der vorangehenden Ansprüche 20-23, das ausgewählt ist aus einem der folgenden Kabel:
- ein Niederspannungskabel, das einen Leiter umgeben von einer Isolationsschicht und optional einer Mantelschicht umfasst, worin die Isolationsschicht eine Polymerzusammensetzung gemäß Anspruch 15 oder 16 umfasst; oder
- ein Stromkabel, das einen elektrischen Leiter umfasst, der von einer oder mehreren Schichten umgeben ist, die wenigstens eine innere halbleitende Schicht, eine Isolationsschicht und eine äußere halbleitende Schicht in dieser Reihenfolge umfassen, und optional von einer Mantelschicht umgeben sind, wobei wenigstens eine der Schichten, vorzugsweise wenigstens die innere halbleitende Schicht und die Isolationsschicht, eine Polymerzusammensetzung gemäß Anspruch 15 oder 16 umfasst.

25. Verfahren zur Herstellung eines vernetzbaren Kabels, umfassend den Schritt des Aufbringens von einer oder mehreren Schichten, die eine Polymerzusammensetzung umfassen, auf einen Leiter, **dadurch gekennzeichnet, dass** in der mindestens einen Schicht eine Polymerzusammensetzung gemäß Anspruch 15 oder 16 verwendet wird.

26. Verfahren nach Anspruch 25 zur Herstellung eines vernetzbaren Kabels gemäß einem der vorangehenden Ansprüche 20-24.

27. Vernetzbares Kabel erhältlich durch ein Verfahren gemäß Anspruch 25 oder Anspruch 26, vorzugsweise ein vernetzbares Niederspannungskabel oder ein vernetzbares Stromkabel.

28. Verfahren zum Vernetzen eines Kabels durch radikalische Reaktion, umfassend:
- das Aufbringen einer oder mehrerer Schichten, die eine Polymerzusammensetzung umfassen, auf einen Leiter, worin zumindest eine Schicht einen oder mehrere Radikalbildner umfasst, und
- das Vernetzen durch radikalische Reaktion der wenigstens einen Schicht, welche freie Radikale erzeugende Mittel umfasst, **dadurch gekennzeichnet, dass** das Vernetzen unter Anwendung eines Verfahrens gemäß Anspruch 17 oder 18 durchgeführt wird.

29. Verfahren nach Anspruch 28 für das Vernetzen eines Kabels, das gemäß Anspruch 25 oder Anspruch 26 hergestellt ist.

30. Verfahren gemäß einem der vorangehenden Ansprüche 28-29, worin das so erhaltene vernetzte Kabel einem weiteren Kühlschritt unterzogen wird, worin dieses vernetzte Kabel unter Druckbedingungen gekühlt wird, und optional das vernetzte und abgekühlte Kabel nach diesem Kühlschritt einem oder mehreren zusätzlichen Schritte unterzogen wird, ausgewählt aus:
- ein druckloser Kühlschritt, worin das vernetzte und abgekühlte Kabel in einem Kühlmedium gekühlt wird,
- ein Rückgewinnungsschritt, worin das vernetzte Kabel nach dem Abkühlungsschritt, vorzugsweise auf eine Kabeltrommel aufgewickelt, gesammelt wird,
- ein Entgasungsschritt, worin der Gehalt an flüchtigen Zersetzungsprodukt(en) reduziert oder entfernt wird, optional bei Raumtemperatur oder bei erhöhter Temperatur, von dem vernetzten Kabel erhalten durch den Kühlungs- und optionalen Rückgewinnungsschritt, und/oder
- ein Fertigstellungsschritt, worin das erhaltene vernetzte Kabel in herkömmlicher Weise zur weiteren Verwendung fertiggestellt wird.

31. Vernetztes Kabel erhältlich durch ein Verfahren gemäß einem der vorangehenden Ansprüche 28-30, vorzugsweise ein vernetztes Niederspannungskabel oder vernetztes Stromkabel.

32. Verwendung einer Verbindung der Formel (I), ausgewählt aus einem oder mehreren von:
- Di-(1-phenyl-cyclohexyl)peroxid (Formel la),
- Di-(1-methyl-1-phenylundecyl)peroxid (Formel Ib), oder
- Di-(1-decyl-1-phenylundecyl)peroxid (Formel Ic),
als ein freie Radikale erzeugendes Mittel in einem Produkt oder einem Verfahren gemäß einem der vorangehenden Ansprüche 14-31, vorzugsweise als Vernetzungsmittel.

## Revendications

1. Composé qui est un peroxyde organique de formule (I) 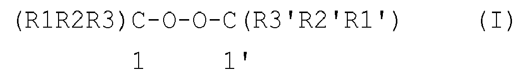 dans laquelle
- R1 et R1' sont chacun indépendamment un hydrocarbyle substitué ou non substitué saturé ou partiellement non saturé ; ou un hydrocarbyle aromatique substitué ou non substitué ;
- dans lequel chacun dudit hydrocarbyle saturé ou partiellement non saturé substitué ou non substitué ou hydrocarbyle aromatique comprend de manière facultative un ou plusieurs hétéroatomes ;
- dans lequel ledit hydrocarbyle saturé ou partiellement non saturé substitué ou non substitué inclut des hydrocarbyles à chaîne linéaire ou ramifiée saturés ou partiellement non saturés ; des hydrocarbyles à chaîne linéaire ou ramifiée saturés ou partiellement non saturés qui portent un hydrocarbyle cyclique saturé ou partiellement non saturé ; et des hydrocarbyles cycliques saturés ou partiellement non saturés ;
- dans lequel chacun dudit hydrocarbyle aromatique et dudit hydrocarbyle cyclique saturé ou partiellement non saturé est indépendamment un système de noyau monocyclique ou multicyclique ; et
- dans lequel ledit hydrocarbyle saturé ou partiellement non saturé substitué ou ledit hydrocarbyle aromatique substitué comprend indépendamment 1 à 4 substituants sélectionnés à partir d'un groupe fonctionnel, un hydrocarbyle saturé ou partiellement non saturé portant de manière facultative un groupe fonctionnel ; ou un hydrocarbyle aromatique portant de manière facultative un groupe fonctionnel ;
- R2, R2', R3 et R3' sont chacun indépendamment H, un hydrocarbyle saturé ou partiellement non saturé substitué ou non substitué ; ou un hydrocarbyle aromatique substitué ou non substitué ;
- dans lequel chacun dudit hydrocarbyle saturé ou partiellement non saturé substitué ou non substitué ou dudit hydrocarbyle aromatique comprend de manière facultative un ou plusieurs hétéroatomes ;
- dans lequel ledit hydrocarbyle saturé ou partiellement non saturé substitué ou non substitué inclut des hydrocarbyles saturés ou partiellement non saturés à chaîne linéaire ou ramifiée ; des hydrocarbyles saturés ou partiellement non saturés à chaîne linéaire ou ramifiée qui portent un hydrocarbyle cyclique saturé ou partiellement non saturé ; et des hydrocarbyles cycliques saturés ou partiellement non saturés ;
- dans lequel chacun dudit hydrocarbyle aromatique et dudit hydrocarbyle cyclique saturé ou partiellement non saturé est indépendamment un système de noyau monocyclique ou multicyclique ; et
- dans lequel ledit hydrocarbyle saturé ou partiellement non saturé substitué ou ledit hydrocarbyle aromatique substitué comprend indépendamment 1 à 4 substituants sélectionnés à partir d'un groupe fonctionnel, un hydrocarbyle saturé ou partiellement non saturé portant de manière facultative un groupe fonctionnel ; ou un hydrocarbyle aromatique portant de manière facultative un groupe fonctionnel ; ou
- R2 et R3 en même temps que l'atome de carbone (C1) auquel ils sont attachés forment une fraction de noyau carbocyclique saturée ou partiellement non saturée substituée ou non substituée de 3 à 14 atomes de C ; une fraction d'hétérocycle saturée ou partiellement non saturée non substituée ou substituée de 3 à 14 atomes de noyau comprenant 1 à 6 hétéroatomes sélectionnés à partir de 0, N, P, S ou Si ; ou une fraction de noyau aromatique non substituée ou substituée de 3 à 14 atomes de C comprenant de manière facultative 1 à 4 hétéroatomes ;
- dans lequel ledit système de noyau aromatique ou d'hétérocycle, de noyau carbocyclique, est fusionné de manière facultative avec un autre système de noyau ayant de 4 à 14 atomes de noyau ; et
- dans lequel ledit système de noyau aromatique ou hétérocycle, de noyau carbocyclique substitué comprend 1 à 4 substituants sélectionnés indépendamment d'un groupe fonctionnel ou d'un hydrocarbyle saturé ou partiellement non saturé portant de manière facultative un groupe fonctionnel ou un hydrocarbyle aromatique portant de manière facultative un groupe fonctionnel ; ou
- R2' et R3' en même temps que l'atome de carbone (C1') auquel ils sont attachés forment une fraction de noyau carbocyclique saturée ou partiellement non saturée non substituée ou substituée de 3 à 14 atomes de C ; une fraction d'hétérocycle saturée ou partiellement non saturée non substituée ou substituée de 3 à 14 atomes de noyau comprenant 1 à 6 hétéroatomes sélectionnés à partir de 0, N, P, S ou Si ; ou une fraction de noyau aromatique non substituée ou substituée de 3 à 14 atomes de C comprenant de manière facultative 1 à 4 hétéroatomes ;
- dans lequel ledit système de noyau aromatique ou hétérocycle, de noyau carbocyclique est fusionné de manière facultative avec un autre système de noyau ayant de 4 à 14 atomes de noyau ; et
- dans lequel ledit système de noyau aromatique ou hétérocycle, de noyau carbocyclique substitué comprend de 1 à 4 substituants sélectionnés indépendamment à partir d'un groupe fonctionnel ou d'un hydrocarbyle saturé ou partiellement non saturé portant de manière facultative un groupe fonctionnel ou d'un hydrocarbyle aromatique portant de manière facultative un groupe fonctionnel ; ou
- R2 et R2' forment ensemble un hydrocarbyle saturé ou en partie non saturé substitué ou non substitué bivalent contenant de manière facultative 1 à 4 hétéroatomes, dans lequel respectivement R2 est lié à C1 et R2' à C1', formant en même temps que -C1-O-O-C1'-une fraction de noyau carbocyclique substituée ou non substituée saturée ou partiellement non saturée de 3 à 14 atomes de C comprenant de manière facultative, en plus desdits au moins deux atomes d'0, 1 à 4 hétéroatomes supplémentaires ; dans lequel ledit système de noyau carbocyclique ou d'hétérocycle est fusionné de manière facultative avec un autre système de noyau ayant de 4 à 14 atomes de noyau ; et des dérivés fonctionnels de ceux-ci ;
- avec la clause restrictive que respectivement au moins deux de R1, R2 et R3 et au moins deux de R1', R2' et R3' sont autres qu'H ou méthyle.

2. Composé de formule (1) selon la revendication 1, dans lequel R2 et R3 en même temps que l'atome de carbone (C1) auquel ils sont attachés forment une fraction de noyau carbocyclique substituée de manière facultative de 3 à 12 atomes de C de noyau ou une fraction d'hétérocycle substituée de manière facultative de 3 à 12 atomes de noyau contenant de 1 à 6, de préférence de 1 à 4, hétéroatomes sélectionnés à partir de 0, N, P, S ou Si, et dans lequel ledit système de noyau carbocyclique ou hétérocyclique est fusionné de manière facultative avec un autre système de noyau ayant de 4 à 14 atomes de noyau, de préférence R2 et R3 en même temps que l'atome de carbone (C1) forment une fraction de noyau carbocyclique (C3-C12) ; de préférence un noyau carbocyclique (C4-C14) mono- ou bicyclique saturé ou partiellement non saturé, substitué de manière facultative, de préférence un noyau carbocyclique (C5-C8) monocyclique saturé non substitué.

3. Composé de formule (1) selon la revendication 1, dans lequel R2' et R3' en même temps que l'atome de carbone (C1') auquel ils sont attachés forment une fraction de noyau carbocyclique substituée de manière facultative de 3 à 12 atomes de C de noyau ou une fraction d'hétérocycle substituée de manière facultative de 3 à 12 atomes de noyau contenant de 1 à 6, de préférence de 1 à 4, hétéroatomes sélectionnés à partir de 0, N, P, S ou Si, et dans lequel ledit système de noyau carbocyclique ou hétérocyclique est fusionné de manière facultative avec un autre système de noyau ayant de 4 à 14 atomes de noyau, de préférence R2 et R3 en même temps que l'atome de carbone (C1') forment une fraction de noyau carbocyclique (C3-C12) ; de préférence un noyau carbocyclique (C4-C14) mono- ou bicyclique saturé ou partiellement non saturé, substitué de manière facultative, de préférence un noyau carbocyclique (C5-C8) monocyclique saturé non substitué.

4. Composé de formule (I) selon l'une quelconque des revendications précédentes 1 à 3, dans lequel R2 et R3 en même temps que l'atome de carbone (C1) auquel ils sont attachés forment un système de noyau selon la revendication 2, et R2' et R3' en même temps que l'atome de carbone (C1') auquel ils sont attachés forment un système de noyau selon la revendication 3, et dans lequel le système de noyau formé par R2' et R3' en même temps que l'atome de carbone (C1') auquel ils sont attachés est le même que le système de noyau formé par R2 et R3 en même temps que l'atome de carbone (C1) auquel ils sont attachés.

5. Composé de formule (I) selon la revendication 1, dans lequel R1, R2, R3, R1' , R2' et R3' sont chacun indépendamment un aryle en (C5-C14) mono- ou multicyclique substitué de manière facultative ; un hétéroaryle en (C5-C14) mono- ou multicyclique substitué de manière facultative ; un cycloalkyle (C4-14) mono- ou multicyclique substitué de manière facultative ; un hétérocyclyle en (C4-C14) mono- ou multicyclique substitué de manière facultative ; un alkyle en (C1-C50) à chaîne linéaire ou ramifiée substitué de manière facultative, de préférence un alkyle en (C1-C30) à chaîne linéaire ; un alcényle en (C1-C50) à chaîne linéaire ou ramifiée substitué de manière facultative ou un alcynyle en (C1-C50) à chaîne linéaire ou ramifiée substitué de manière facultative, de préférence un alcényle en (C1-C30) à chaîne linéaire ou un alcynyle en (C1-C30) à chaîne linéaire ; un hétéroalkyle en (C1-C50) à chaîne linéaire ou ramifiée substitué de manière facultative comprenant 1 à 4 hétéroatomes sélectionnés à partir de 0, N, P, S ou Si ; de préférence R2, R2', R3 et R3' sont indépendamment sélectionnés à partir d'un alkyle en (C1-C50) à chaîne linéaire non substitué, de préférence un alkyle en (C1-C30), plus de préférence un alkyle en (C1-C20).

6. Composé de formule (I) selon les revendications 1 à 5, dans lequel R2 et R2' représentent chacun le même radical et, respectivement, R3 et R3' représentent chacun le même radical.

7. Composé de formule (I) selon la revendication 1, 5 ou 6, dans lequel
- R2 et R2' sont les mêmes et représentent chacun du méthyle ; ou
- R2 et R2' sont les mêmes et représentent chacun un alkyle en (C6-C30).

8. Composé de formule (I) selon la revendication 1 ou 5 à 7, dans lequel R3 et R3' sont les mêmes et représentent chacun un alkyle en (C6-C30).

9. Composé de formule (I) selon l'une quelconque des revendications précédentes 1 à 8, dans lequel
- R1 et R1' sont les mêmes ou différents, de préférence les mêmes, et représentent chacun un hydrocarbyle cyclique, saturé ou partiellement non saturé, substitué de manière facultative, de 5 à 14 atomes de noyau contenant de manière facultative 1 à 4 atomes d'hétérocycle sélectionnés à partir de N, 0, P, S ou Si ; ou un aryle en (C5-C14) mono- ou multicyclique substitué de manière facultative, de préférence un aryle en (C5-C7) monocyclique non substitué ; ou
- R1 et R1' sont les mêmes ou différents, de préférence les mêmes, et représentent chacun un méthyle ou un alkyle en (C6-C30) à chaîne linéaire ou ramifiée, substitué de manière facultative, de préférence à chaîne linéaire non substitué.

10. Composé de formule (I) selon l'une quelconque des revendications précédentes 1 à 9, dans lequel lorsque plus d'un de R1, R2, R3 est un alkyle selon l'une quelconque des revendications précédentes 1 ou 5 à 9 et un de R1, R2, R3 comme ledit alkyle est du méthyle, de préférence un alkyle en (C1-C3), alors le reste de R1, R2 et R3 comme ledit alkyle représente indépendamment un alkyle en (C6-C20) ou, respectivement, lorsque plus d'un de R1', R2', R3' est un alkyle selon l'une quelconque des revendications précédentes 1 ou 5 à 9 et un de R1', R2', R3' comme ledit alkyle est du méthyle, alors le reste de R1', R2' et R3' comme ledit alkyle représente indépendamment un alkyle en (C6-C20).

11. Composé de formule (I) selon la revendication 1, qui est sélectionnée à partir de n'importe lequel de
- Di-(1-méthyl-1-phénylundécyl) peroxyde (formule Ib), ou
- Di-(1-décyl-1-phénylundécyl) peroxyde (formule Ic).

12. Utilisation d'un composé selon l'une quelconque des revendications 1 à 11 en tant que radical libre produisant un agent pour modifier des polymères.

13. Utilisation d'un composé selon la revendication 12, sélectionnée à partir des usages suivants :
(1) réticulation de polymères via une réaction par radicaux ;
(2) greffe de polymères avec des composés, de préférence des composés non saturés, via une réaction par radicaux ; ou
(3) viscoréduction d'un polymère pour modifier l'indice de fluidité (MFR) dudit polymère via une réaction par radicaux ;
de préférence pour (1) réticulation de polymères.

14. Utilisation d'un composé selon la revendication 12 ou 13 pour modifier des polymères dans des applications de fil et de câble.

15. Composition de polymère comprenant un composé selon l'une quelconque des revendications 1 à 11.

16. Composition de polymère selon la revendication 15 qui est sous forme (1) d'une poudre de polymère, (2) de pastilles de polymère ou (3) d'un mélange d'une fusion de polymère,
de sorte que ladite (1) poudre de polymère ou, de préférence, lesdites (2) pastilles de polymère sont contenues de manière facultative dans un conteneur.

17. Processus pour réticuler une composition de polymère par une réaction par radicaux en utilisant un ou plusieurs agents de production de radical libre **caractérisé en ce que** la réticulation est effectuée en utilisant un agent de production de radical libre qui est un composé selon l'une quelconque des revendications 1 à 11.

18. Processus selon la revendication 17, **caractérisé en ce que** la réticulation est effectuée en produisant du méthane en tant que produit de décomposition de ladite étape de réticulation en une quantité inférieure à 300 ppm (poids), quand on le détermine à partir de l'échantillon de polymère réticulé produit selon un procédé tel que décrit dans la description sous « protocole d'analyse GC », de préférence ladite étape de réticulation est effectuée sans produire de méthane en tant que produit de décomposition de ladite étape de réticulation.

19. Composition réticulée que l'on peut obtenir par un processus de réticulation selon la revendication 17 comprenant du CH4 en une quantité inférieure à 300 ppm (poids), de préférence inférieure à 200 ppm (poids), de façon appropriée inférieure à 100 ppm (poids), de préférence en une quantité de 0 à 50 ppm (poids), en tant que produit de décomposition de ladite étape de réticulation, quand on le détermine à partir de l'échantillon de composition réticulée selon un procédé tel que décrit dans la description sous « protocole d'analyse GC »,

20. Câble pouvant être réticulé qui comprend un conducteur qui est entouré par une ou plusieurs couches comprenant une composition de polymère, **caractérisé en ce qu'**au moins une couche comprend une composition de polymère selon la revendication 15 ou 16.

21. Câble pouvant être réticulé selon la revendication 20, qui comprend au moins une couche isolante qui comprend une composition de polymère selon la revendication 15 ou 16.

22. Câble pouvant être réticulé selon la revendication 20 ou 21, qui comprend au moins une couche semi-conductrice comprenant une composition de polymère selon la revendication 15 ou 16.

23. Câble pouvant être réticulé selon la revendication 20, 21 ou 22, qui comprend une couche de gainage et de manière facultative une ou plusieurs couches sélectionnées à partir d'une couche isolante et d'une couche semi-conductrice entourée par ladite couche de gainage, dans lequel ladite couche de gainage comprend une composition de polymère selon la revendication 15 ou 16.

24. Câble pouvant être réticulé selon l'une quelconque des revendications précédentes 20 à 23, qui est sélectionné à partir de n'importe lequel des câbles suivants :
- un câble basse tension comprenant un conducteur entouré par une couche isolante et de manière facultative une couche de gainage, dans lequel ladite couche isolante comprend une composition de polymère selon la revendication 15 ou 16 ; ou
- un câble de puissance comprenant un conducteur électrique entouré par une ou plusieurs couches comprenant au moins une couche semi-conductrice intérieure, une couche isolante et une couche semi-conductrice extérieure, dans cet ordre, et entouré de manière facultative par une couche de gainage, dans lequel au moins une desdites couches comprend, de préférence au moins une couche semi-conductrice intérieure et une couche isolante, une composition de polymère selon la revendication 15 ou 16.

25. Processus pour produire un câble pouvant être réticulé comprenant une étape d'application d'une ou plusieurs couches comprenant une composition de polymère sur un conducteur, **caractérisé en ce que** dans ladite au moins une couche on utilise une composition de polymère selon la revendication 15 ou 16.

26. Processus selon la revendication 25, pour préparer un câble pouvant être réticulé selon l'une quelconque des revendications précédentes 20 à 24.

27. Câble pouvant être réticulé que l'on peut obtenir par un processus selon la revendication 25 ou la revendication 26, de préférence un câble basse tension pouvant être réticulé ou un câble de puissance pouvant être réticulé.

28. Processus pour réticuler un câble par une réaction par radicaux, comprenant :
- l'application d'une ou de plusieurs couches comprenant une composition de polymère sur un conducteur, dans lequel au moins une couche comprend un ou plusieurs agents de production de radical libre, et
- la réticulation par une réaction par radicaux de ladite au moins une couche comprenant le(s)dit(s) agent(s) de production de radical libre, **caractérisée en ce que** ladite réticulation est effectuée en utilisant un processus selon la revendication 17 ou 18.

29. Processus selon la revendication 28, pour réticuler un câble qui est préparé selon la revendication 25 ou la revendication 26.

30. Processus selon l'une quelconque des revendications précédentes 28 et 29, dans lequel le câble réticulé ainsi obtenu est soumis à une étape de refroidissement supplémentaire, dans laquelle ledit câble réticulé est refroidi sous des conditions pressurisées,
et, de manière facultative, après ladite étape de refroidissement, le câble réticulé et refroidi est soumis à une ou plusieurs étapes supplémentaires sélectionnées à partir de :
- une étape de refroidissement non pressurisé, dans lequel le câble réticulé et refroidi est en outre refroidi dans un milieu de refroidissement,
- une étape de récupération, dans laquelle le câble réticulé est recueilli après l'étape de refroidissement, de préférence enroulé sur un tambour de câble,
- une étape de dégazage, dans laquelle la teneur en produits(s) de décomposition volatil(s) est réduite ou supprimée, de manière facultative à température ambiante ou sous une température élevée, depuis ledit câble réticulé obtenu à partir de ladite étape de refroidissement et de récupération facultative, et/ou
- une étape de finition, dans laquelle le câble réticulé obtenu est fini d'une façon classique pour une utilisation ultérieure.

31. Câble réticulé que l'on peut obtenir par un processus selon l'une quelconque des revendications précédentes 28 à 30, de préférence un câble basse tension réticulé ou un câble de puissance réticulé.

32. Utilisation d'un composé de formule (I) sélectionné à partir d'un ou plusieurs de :
- Di-(1-phénylcyclohexyl) peroxyde (formule Ia),
- Di-(1-méthyl-1-phénylundécyl) peroxyde (formule Ib), ou
- Di-(1-décyl- 1-phénylundécyl) peroxyde (formule Ic) ,
en tant qu'agent de production de radical libre dans un produit ou un processus selon l'une quelconque des revendications précédentes 14 à 31, de préférence en tant qu'agent de réticulation.
